# EUROPEAN PATENT APPLICATION

(11) **EP 4 663 659 A1**
(43) Date of publication of application: **17.12.2025**
(21) Application number: 24181259.3
(22) Date of filing: 10.06.2024
(51) Int. Cl.: C07K 16/28, A61P 35/00, C07K 16/30, A61K 39/00

(54) **MUC-1 CONDITIONAL CD40 AGONISTS**

(71) Applicant: Merck Patent GmbH, 64293 Darmstadt (DE)
(72) Inventor: SHA, Weixiao, 64291 Darmstadt (DE); GUENTHER, Ralf, 64347 Griesheim (DE); ZIELONKA, Stefan, 64297 Darmstadt (DE); KRAH, Simon, 64289 Darmstadt (DE)
(74) Representative: Merck Patent Association

(57) **Abstract**

The present invention provides bispecific binding proteins binding MUC-1 and CD40 and pharmaceutical compositions, and their use to upregulate cell-mediated immune responses and for the treatment of cancer.

## Description

### TECHNICAL FIELD

The present application relates to bispecific binding proteins binding MUC-1 and CD40 such as antibodies or antigen binding fragments thereof, nucleic acids encoding the same, therapeutic compositions thereof, and their use to enhance T-cell function, to upregulate cell-mediated immune responses and for the treatment of T cell dysfunctional disorders and cancer.

### BACKGROUND OF THE INVENTION

### CD40 signaling

The induction of an adaptive immune response requires several different molecular signals. As primary signal, binding of an antigen to an antigen receptor expressed by T and B lymphocytes is required. Subsequent secondary signals involve costimulatory receptor-ligand interactions between T cells and antigen-presenting cells (APCs) and between B cells and T cells, and the secretion of cytokines.

One of these co-stimulatory receptors providing a secondary signal is CD40 (cluster of differentiation 40), a member of the tumor necrosis factor receptor (TNFR) family. CD40 is a type I transmembrane protein that is expressed by B cells and professional antigen-expressing cells, but also by tumor cells. The ligand of CD40 is CD40L (CD154) which is expressed by activated T cells, activated B cells and platelets as well as, under inflammatory conditions, some other immune cell types. CD40 acts as a stimulator of the immune system and regulates a variety of processes involved in cellular and humoral adaptive immunity. For example, the interaction between CD40 and CD40L promotes the maturation of dendritic cells, enhances the antigen-presenting capability, and facilitates T-cell activation and differentiation. In B cells, engagement with CD40L leads to germinal center formation, immunoglobulin isotype switching, somatic hypermutation, and the development of memory B cells and long-lived plasma cells.

The CD40/CD40L interaction has been studied with regard to its implications in protective anti-tumor immunity. Anti-CD40L monoclonal antibody treatment has been shown to inhibit protective immune responses and to block efficacy of potent tumor vaccines and these results were confirmed using CD40-deficient mice. Conversely, CD40 agonistic antibodies have been shown to stimulate anti-tumor immunity. Such antibodies elicit secretion of various cytokines and upregulation of co-stimulatory molecules. A combination of a CD40 agonist and a TLR (Toll-like receptor) agonist has shown therapeutic efficacy in various tumor models.

Human CD40 monoclonal antibodies have been developed and tested in clinical trials due to effects shown in cancer animal models. However, due to toxicities observed, administration is limited to suboptimal doses resulting in only low immune responses (Salomon R and Dahan R, Front Immunol. 2022 Jul 13:13:940674. doi: 10.3389/fimmu.2022.940674. eCollection 2022).

For detailed review of CD40 and CD40-based immunotherapic approaches in cancer, see Elgueta et al., Immunol Rev. 2009 May; 229(1): 10.1111/j.1600-065X.2009.00782.x and references cited therein.

### MUC-1 signaling

MUC-1 (mucin 1; also referred to as MUC1) is a type I transmembrane glycoprotein of the mucin family of proteins that acts in normal cells as a lubricant, moisturizer, and physical barrier. MUC-1 is present on the surface of epithelial cells in a tight network forming a protective barrier and protecting cells from their surroundings and pathogen infections. In cancer cells, MUC-1 is overexpressed, exhibits aberrant glycosylation and is involved in cancer invasion, metastasis, angiogenesis, and apoptosis. MUC1 is an oncogene that can play a pro- or anti-inflammatory role in different infection-induced cancers by acting as an immunomodulatory switch, supports progression from inflammation into cancer, mediates drug resistance by blocking cell access of chemotherapy drugs and promotes tumor metastasis. MUC-1 is overexpressed in various cancer types including lung, liver, colon, breast, pancreatic, and ovarian cancer. The protein is synthesized in the rough endoplasmic reticulum as one large precursor and subsequently proteolytically cleaved into two fragments, a cytoplasmic domain (including the transmembrane domain) and an extracellular domain. These fragments assemble into a very stable complex in a non-covalent fashion (Ligtenberg MJ et al., The Journal of Biological Chemistry 1992 March 25; 267(9):6171-6177).

For cancer therapy, MUC-1-targeting therapeutic approaches have been investigated, including vaccines directed against MUC-1 overexpression and MUC-1-based immunotherapeutic strategies.

For detailed review of MUC-1 structure, function and clinical applications see Chen W et al., Int J Mol Sci. 2021;22:6567; https://doi.org/10.3390/ijms22126567 and references cited therein.

Immunotherapies such as immune checkpoint inhibition (ICI) have provided durable responses and benefit to cancer patients. However, only a fraction of patients responds to ICI, potentially due to a lack of activating stimuli for the immune system. First generation CD40 agonists induce promising anti-tumor immune responses but their dosing is limited due to on-target systemic toxicities in both preclinical and clinical settings. Approaches to broaden the therapeutic window of CD40 agonists are needed to provide new options as standard of care for cancer patients.

### SUMMARY OF THE INVENTION

The present invention provides a bispecific binding protein binding to MUC-1 and CD40. The bispecific binding protein of the present invention can be used in the treatment of cancer or a T cell dysfunctional disorder.

The bispecific binding protein of the present invention activates CD40 in the presence of MUC-1 tumor antigen, which is highly overexpressed in many tumor types. The bispecific binding protein of the present invention induces a higher magnitude of CD40 modulation than first generation anti-CD40 antibodies (e.g., selicrelumab, sotigalimab) in the presence of MUC-1 expressing tumor cells, while there is no activity of the bispecific binding protein of the present invention in the absence of MUC-1 expressing tumor cells. Normal tissues such as normal kidney and lung cells activate CD40 to a lower extent than MUC-1 expressing tumor cells. The minimized MUC-1-independent CD40 activation and low activity of the bispecific binding protein of the present invention using normal tissue cells reduces off-tumor toxicity and broadens the therapeutic window of CD40 activation.

### BRIEF DESCRIPTION OF THE DRAWINGS

Figure 1: *In vitro* activity of MUC-1 conditional CD40 agonist is superior towards clinical stage anti-CD40 benchmark antibodies (selicrelumab, sotigalimab). Monocyte-derived dendritic cells were co-cultured with MUC-1 expressing HCC827 tumor cells, and IL-12 (panel A: IL12p70; panel B: IL12p40) was measured via ELISA (enzyme-linked immunosorbent assay) after 24 hrs.
Figure 2: No activation of CD40 in the absence of MUC-1 expressing tumor cells. Monocyte-derived dendritic cells were cultured without HCC827 tumor cells, and IL-12 (panel A: IL12p40; panel B: IL12p70) was measured via ELISA after 24 hrs.
Figure 3: Magnitude of B cell activation depends on anti-MUC-1 binding to tumor cells. Various human tumor cell lines were co-cultured with freshly isolated B cells and CD86 positive cells were detected via flow cytometry. MUC-1 surface expression was quantified by anti-MUC-1 cellular binding measured via flow cytometry.
Figure 4: MUC-1 conditional agonist targeting human CD40 only induces IL-12p40 in immature Dendritic Cells (iDCs) (Figure 4A) but not M-CSF (macrophage colony-stimulating factor) macrophages (Figure 4B). Monocyte-derived dendritic cells or monocyte-derived M-CSF macrophages were co-cultured with MUC-1 expressing HCC827 tumor cells and IL-12 measured via ELISA after 24hrs.
Figure 5: Kidney and Lung express high levels of MUC-1. Tissue micro arrays from healthy tissues (FDA normal panel) were stained with anti-MUC-1 IHC (immunohistochemistry) antibody and MUC-1 staining was quantified and IHC H-Score assessed.
Figure 6: MUC-1 expression on tumor cell lines and normal tissue cell lines. Tumor cells and normal tissue cells were cultured *in vitro* and MUC-1 surface expression was quantified by anti-MUC-1 cellular binding measured via flow cytometry.
Figure 7: Low activation of CD40 by anti MUC-1 × CD40 using normal tissue Kidney (Figure 7A) and Lung (Figure 7B). Monocyte-derived dendritic cells were co-cultured with MUC-1 expressing kidney and lung normal cells, and CD80/CD86 double positive dendritic cells were measured via flow cytometry after 24hrs.
Figure 8: *In vitro* activity of Mouse Surrogate anti MUC-1 × mCD40. Mouse B cells were co-cultured with MUC-1 expressing MC38 tumor cells and B cell activation was measured via quantification of CD86+ B cells using flow cytometry.
Figure 9: *In vivo* activity of Mouse Surrogate anti MUC-1 × mCD40 in MUC-1-MC38 tumor model. MUC-1-MC38 tumor cells were inoculated s.c. into mice and treated with either isotype control antibody or Mouse Surrogate anti-MUC-1 × mCD40. Tumor size after treatment was assessed using a caliper.
Figure 10: *In vivo* activity of Mouse Surrogate anti MUC-1 × mCD40 in MUC-1-Panc02 tumor model (individual tumor growth). MUC-1-Panc02 tumor cells were orthotopically transplanted into mice via surgery and treated with either Vehicle control (bottom panel) or Mouse Surrogate anti-MUC-1 × mCD40 (top panel). Tumor size after treatment was assessed using a caliper.
Figure 11: Mouse Surrogate anti MUC-1 × mCD40 does not induce body weight loss in mice bearing MUC-1 expressing WISH tumor model (top panel) and MUC-1-MC38 tumor model (bottom panel). Tumor cells were inoculated s.c. into mice and body weight monitored after treatment with anti-MUC-1 × mCD40 or anti-mCD40.

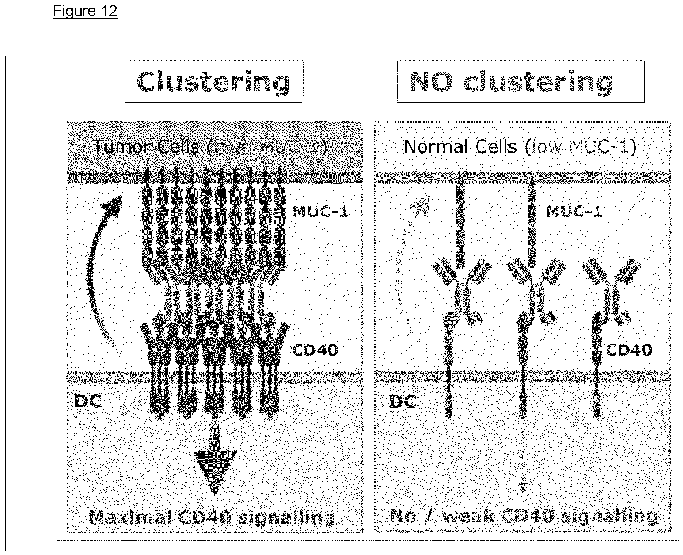

Figure 12: Chart of comparison of normal tissue versus tumor tissue treated with bispecific anti MUC-1 × CD40 antibodies. Clustering of CD40 parallel by enhanced CD40 signalling in DC cells triggered by tumor cells (right side), no clustering in normal cells (left side).

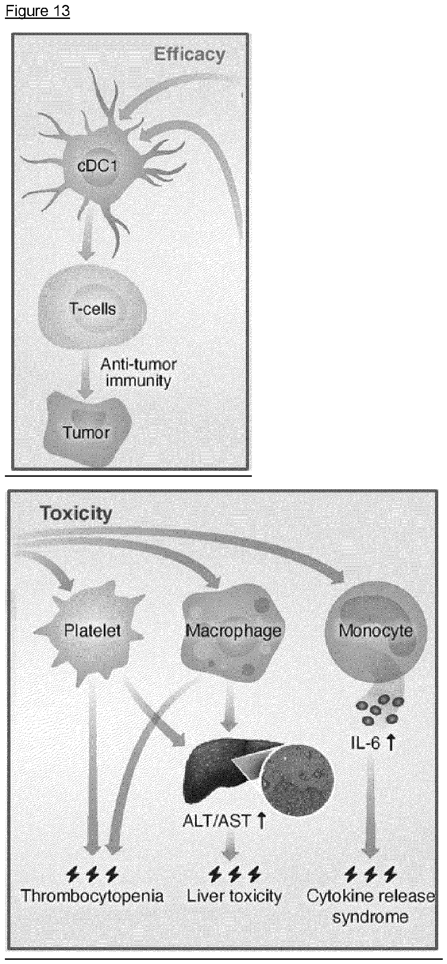

Figure 13: Chart of comparison of Tumor-specific action (Efficacy, upper panel) and systemic CD40 activation (Toxicity, lower panel).

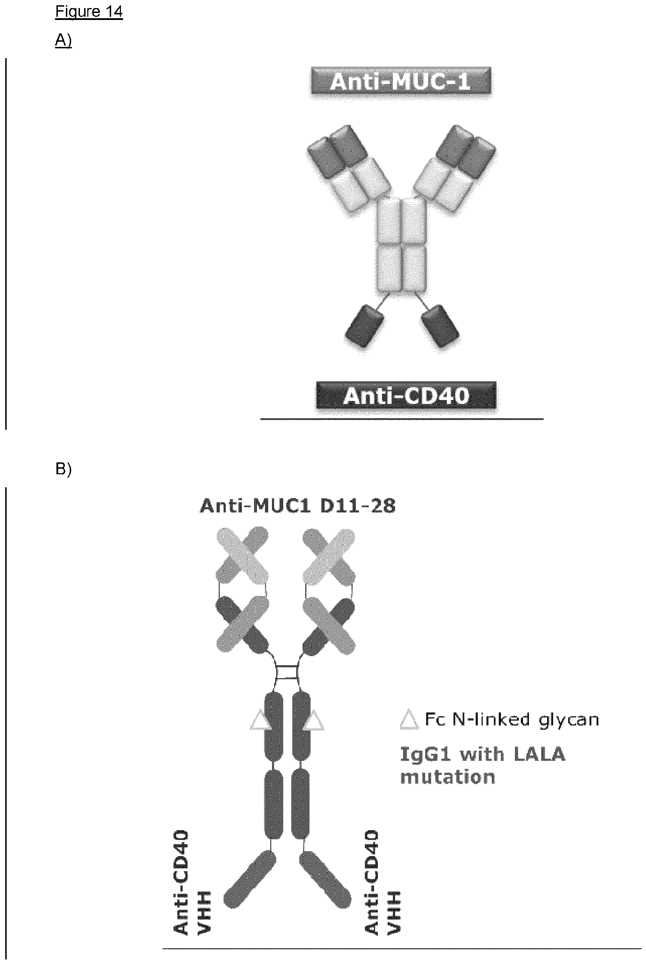

Figure 14: A: Schematic cartoon of anti-MUC-1 × anti-CD40 (i.e., the bispecific binding protein FP1). B: More detailed schematic cartoon of anti-MUC-1 × anti-CD40. FP1 is a bispecific fusion protein comprising two anti-human CD40 VHH domains (SEQ ID NO: 15) covalently linked via a flexible 15aa GS-linkers (SEQ ID NO: 11) to the C-terminus of the heavy chains of a fully human anti-MUC-1 IgG1 antibody (heavy chain VH: SEQ ID NO: 4; light chain VL: SEQ ID NO: 9). The first anti-CD40 VHH is linked via GS-linker to the C-terminus of the first heavy chain of the anti-MUC-1 IgG1 antibody, the second anti-CD40 VHH is linked via GS-linker to the C-terminus of the second heavy chain of the anti-MUC-1 IgG1 antibody. This results, preferably, in an IgG1 fusion protein with two heavy chains (in each case SEQ ID NO: 16, which comprises the anti-MUC-1 IgG1 heavy chain sequence, the GS-linker sequence and the anti-CD40 VHH sequence) associated with two light chains (SEQ ID NO: 10), wherein the two heavy chains are linked by two IgG1 interchain disulfide bonds. The format of the molecule enables conditional activation of CD40 on antigen-presenting cells to kick-start T-cell response.

### DETAILED DESCRIPTION OF THE INVENTION

Although the present disclosure is described in detail above and below, it is to be understood that this disclosure is not limited to the particular methodologies, protocols and reagents described by the present disclosure, as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

In the following, certain elements of the present disclosure will be described in more detail, including the description of specific embodiments. However, the variously described examples and preferred embodiments should not be construed to limit the present disclosure to only the explicitly described embodiments. This description should be understood to support and encompass embodiments which combine the explicitly described embodiments with any number of the disclosed and/or preferred elements and in any manner. Furthermore, any permutations and combinations of all described elements in this application should be considered disclosed by the description of the present application except for where this leads to logical contradictions or the context indicates otherwise.

Unless defined otherwise herein, scientific and technical terms used in connection with the present disclosure shall have the meanings that are commonly understood by those of ordinary skill in the art. Generally, nomenclatures and techniques referred to in the present disclosure, e.g. nomenclatures and techniques of biology, medicinal and pharmaceutical chemistry, medicine, pharmacology, toxicology, organic chemistry or chemical synthesis, are those well-known and commonly used in the art. The methods and techniques of the present disclosure are generally performed according to conventional methods well-known in the art and as described in the references cited and discussed throughout the present disclosure unless otherwise indicated.

### Definitions

"Antibodies", also called "immunoglobulins" (Ig), generally comprise four polypeptide chains, two heavy (H) chains and two light (L) chains, and are therefore multimeric proteins. The term "antibodies" also includes full length functional mutants, variants, or derivatives thereof (including, but not limited to, murine, chimeric, humanized and fully human antibodies) which retain the essential epitope binding features of an Ig molecule, and includes dual specific, bispecific, multispecific, and dual variable domain Igs. Ig molecules can be of any class (e.g., IgG, IgE, IgM, IgD, IgA, and IgY), or subclass (e.g., IgG1, IgG2, IgG3, IgG4, IgA1, and IgA2) and allotype. Ig molecules may also be mutated e.g. to enhance or reduce affinity for Fcγ receptors or the neonatal Fc receptor (FcRn).

The term "LALA mutation" or "lgG1 LALA mutation" used herein refers to a L234A/L235A (LALA) double mutated IgG1 variant. The LALA mutation was first described by Lund et al. 1991, J Immunol. 147:2657-2662. The mutation leads to reduced binding to the IgG Fc receptors FcγRI, FcγRII and FcγRIII.

A monoclonal antibody may be obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site or determinant on the antigen, in contrast to conventional (polyclonal) antibody preparations which typically include different antibodies directed against different determinants (or epitopes). In addition to their specificity, the monoclonal antibodies are advantageous in that they are synthesized by the hybridoma culture, hence uncontaminated by other immunoglobulins. The modifier "monoclonal" indicates the character of the antibody as being obtained from a substantially homogeneous population of antibodies, and is not to be construed as requiring production of the antibody by any particular method.

For the preparation of monoclonal antibodies, any technique providing antibodies produced by continuous cell line cultures can be used. For example, monoclonal antibodies to be used may be made by the hybridoma method first described by Koehler et al., Nature, 256: 495 (1975), or may be made by recombinant DNA methods (see, e.g., U.S. Patent No. 4,816,567). Examples for further techniques to produce human monoclonal antibodies include the trioma technique, the human B-cell hybridoma technique (Kozbor, Immunology Today 4 (1983), 72) and the EBV- hybridoma technique (Cole et al., Monoclonal Antibodies and Cancer Therapy, Alan R. Liss, Inc. (1985), 77-96).

Hybridomas can then be screened using standard methods, such as enzyme-linked immunosorbent assay (ELISA) and surface plasmon resonance (BIACORE^{™}) analysis, to identify one or more hybridomas that produce an antibody that specifically binds with a specified antigen. Any form of the relevant antigen may be used as the immunogen, e.g., recombinant antigen, naturally occurring forms, any variants or fragments thereof, as well as an antigenic peptide thereof. Surface plasmon resonance as employed in the BIAcore system can be used to increase the efficiency of phage antibodies which bind to an epitope of a target antigen (Schier, Human Antibodies Hybridomas 7 (1996), 97-105; Malmborg, J. Immunol. Methods 183 (1995), 7-13). Another exemplary method of making monoclonal antibodies includes screening protein expression libraries, e.g., phage display or ribosome display libraries. Phage display is described, for example, in Ladner et al., U.S. Patent No. 5,223,409; Smith (1985) Science 228:1315-1317, Clackson et al., Nature, 352: 624-628 (1991 ) and Marks et al., J. Mol. Biol., 222: 581 -597 (1991).

In addition to the use of display libraries, the relevant antigen can be used to immunize a non- human animal, e.g., a rodent (such as a mouse, hamster, rabbit or rat). In one embodiment, the non-human animal includes at least a part of a human immunoglobulin gene. For example, it is possible to engineer mouse strains deficient in mouse antibody production with large fragments of the human Ig (immunoglobulin) loci. Using the hybridoma technology, antigen-specific monoclonal antibodies derived from the genes with the desired specificity may be produced and selected. See, e.g., XENOMOUSE™, Green et al. (1994) Nature Genetics 7:13-21 , US 2003- 0070185, WO 96/34096, and WO 96/33735.

A monoclonal antibody can also be obtained from a non-human animal, and then modified, e.g., humanized, deimmunized, rendered chimeric etc., using recombinant DNA techniques known in the art. Examples of modified antibody constructs include humanized variants of non-human antibodies, "affinity matured" antibodies (see, e.g. Hawkins et al. J. Mol. Biol. 254, 889-896 (1992) and Lowman et al., Biochemistry 30, 10832- 10837 (1991 )) and antibody mutants with altered effector function(s) (see, e.g., US Patent 5,648,260, Kontermann and Dubel (2010), loc. cit. and Little (2009), loc. cit).

In immunology, affinity maturation is the process by which B cells produce antibodies with increased affinity for antigen during the course of an immune response. With repeated exposures to the same antigen, a host will produce antibodies of successively greater affinities. Like the natural prototype, the in vitro affinity maturation is based on the principles of mutation and selection. The in vitro affinity maturation has successfully been used to optimize antibodies, antibody constructs, and antibody fragments. Random mutations inside the CDRs are introduced using radiation, chemical mutagens or error-prone PCR. In addition, genetical diversity can be increased by chain shuffling. Two or three rounds of mutation and selection using display methods like phage display usually results in antibody fragments with affinities in the low nanomolar range. A preferred type of an amino acid substitutional variation of the antibody constructs involves substituting one or more hypervariable region residues of a parent antibody (e. g. a humanized or human antibody). Generally, the resulting variant(s) selected for further development will have improved biological properties relative to the parent antibody from which they are generated. A convenient way for generating such substitutional variants involves affinity maturation using phage display. Alternatively, or additionally, it may be beneficial to analyze a crystal structure of the antigen-antibody complex to identify contact points between the binding domain and its target. Such contact residues and neighboring residues are candidates for substitution according to the techniques elaborated herein. Once such variants are generated, the panel of variants is subjected to screening as described herein and antibodies with superior properties in one or more relevant assays may be selected for further development.

An "isolated" antibody is one that has been identified, separated and/or recovered from a component of its production environment (e.g., natural or recombinant). Preferably, the isolated polypeptide is free of association with all other components from its production environment. Contaminant components of its production environment, such as that resulting from recombinant transfected cells, are materials that would typically interfere with research, diagnostic or therapeutic uses for the antibody, and may include enzymes, hormones, and other proteinaceous or non-proteinaceous solutes. In preferred embodiments, the polypeptide will be purified: (i) to greater than 95% by weight of antibody as determined by, for example, the Lowry method, and in some embodiments, to greater than 99% by weight; or (ii) to homogeneity by SDS-PAGE under non-reducing or reducing conditions using Coomassie blue or, preferably, silver stain. Isolated antibody includes the antibody *in situ* within recombinant cells since at least one component of the antibody's natural environment will not be present. Ordinarily, however, an isolated polypeptide or antibody will be prepared by at least one purification step.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies (see Kabat et al, Sequences of Immunological Interest, Fifth Edition, National Institute of Health, Bethesda, MD (1991)). The constant domains are not involved directly in the binding of antibody to an antigen, but exhibit various effector functions, such as participation of the antibody in antibody-dependent cellular toxicity.

An "antibody fragment" relates to a molecule comprising at least one polypeptide chain derived from an antibody that is not full length and exhibits target binding. Antibody fragments are capable of binding to the same epitope or target as their corresponding full-length antibody. Antibody fragments include, but are not limited to (i) a Fab fragment, which is a monovalent fragment consisting of the variable light (VL), variable heavy (VH), constant light (CL) and constant heavy 1 (CH1) domains; (ii) a F(ab')₂ fragment, which is a bivalent fragment comprising two Fab fragments linked by a disulfide bridge at the hinge region (reduction of a F(ab')₂ fragment result in two Fab' fragment with a free sulfhydryl group); (iii) a heavy chain portion of a Fab (Fa) fragment, which consists of the VH and CH1 domains; (iv) a variable fragment (Fv) fragment, which consists of the VL and VH domains of a single arm of an antibody; (v) a domain antibody (dAb) fragment, which comprises a single variable domain; (vi) an isolated complementarity determining region (CDR); (vii) a single chain Fv fragment (scFv); (viii) a diabody, which is a bivalent, bispecific antibody in which VH and VL domains are expressed on a single polypeptide chain, but using a linker that is too short to allow for pairing between the two domains on the same chain, thereby forcing the domains to pair with the complementarity domains of another chain and creating two antigen binding sites; (ix) a linear antibody, which comprises a pair of tandem Fv segments (VH-CH1-VH-CH1) which, together with complementarity light chain polypeptides, form a pair of antigen binding regions; (x) Dual-Variable Domain Immunoglobulin; (xi) other non-full length portions of immunoglobulin heavy and/or light chains, or mutants, variants, or derivatives thereof, alone or in any combination.

The term "VHH" or "nanobody"^{®} refers to single-domain antibodies which are antibody fragments consisting of a single monomeric variable antibody domain. Like a whole antibody, they are able to bind selectively to a specific antigen. With a molecular weight of only 12-15 kDa, single-domain antibodies are much smaller than common antibodies (150-160 kDa). The first single-domain antibodies were engineered from heavy-chain antibodies found in camelids (Gibbs, W. Wayt (August 2005). "Nanobodies". Scientific American Magazine)

"Anti MUC-1 × CD40" is synonymous with "anti-MUC1 × anti-CD40", "anti MUC-1 × hCD40 A02" and "Fusion protein 1" (abbreviated "FP1") and refers to the bispecific binding protein according to the present disclosure as described e.g. in Example 1 and Figure 14.

As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In some embodiments, the extent of binding of an antibody to an unrelated target is less than about 10% of the binding of the antibody to the target as measured, e.g., by a radioimmunoassay (RIA). In certain embodiments, an antibody that specifically binds to a target has a dissociation constant (KD) of < 1×10⁻⁶M, < 1×10⁻⁷M, < 1×10⁻⁸M, < 1×10⁻⁹M, or < 1×10⁻¹⁰M. In certain embodiments, an antibody specifically binds to an epitope on a protein that is conserved among the protein from different species. In another embodiment, specific binding can include, but does not require, exclusive binding.

"Binding affinity" generally refers to the strength of the sum total of non-covalent interactions between a single binding site of a molecule (e.g., of an antibody) and its binding partner (e.g., an antigen). Unless indicated otherwise, as used herein, "binding affinity", "bind to", "binds to" or "binding to" refers to intrinsic binding affinity that reflects a 1:1 interaction between members of a binding pair (e.g., antibody Fab fragment and antigen). The affinity of a molecule X for its partner Y can generally be represented by the dissociation constant (KD). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

The "KD" or "KD value" according to this invention is in some embodiments measured by a radiolabelled antigen binding assay (RIA) performed, e.g., with the Fab version of the antibody and antigen molecule that measures solution binding affinity of Fabs for antigen by equilibrating Fab with a minimal concentration of (1251)-labelled antigen in the presence of a titration series of unlabelled antigen, then capturing bound antigen with an anti- Fab antibody-coated plate (Chen, et al, (1999) J. Mol Biol 293:865-881). According to another embodiment, the KD is measured by using surface-plasmon resonance assays using a BIACORE^{®}-2000 or a BIACORE^{®}-3000 instrument (BIAcore, Inc., Piscataway, NJ). The equilibrium dissociation constant (KD) is calculated as the ratio koff/kon (e.g., Chen et al, J. Mol. Biol. 293:865-881 (1999)).

"Antibody-dependent cell-mediated cytotoxicity" or ADCC refers to a form of cytotoxicity in which secreted Ig bound onto Fc receptors (FcRs) present on certain cytotoxic cells (e.g., natural killer (NK) cells, neutrophils and macrophages) enable these cytotoxic effector cells to bind specifically to an antigen-bearing target cell and subsequently kill the target cell with cytotoxins. The antibodies "arm" the cytotoxic cells and are required for killing of the target cell by this mechanism. The primary cells for mediating ADCC, NK cells, express FcγRIII only, whereas monocytes express FcγRI, FcγRII and FcγRIII. Fc expression on hematopoietic cells is summarized in Table 3 on page 464 of Ravetch and Kinet, Annu. Rev. Immunol. 9: 457-92 (1991). To assess ADCC activity of a molecule of interest, an *in vitro* ADCC assay. Useful effector cells for such assays include peripheral blood mononuclear cells (PBMC) and natural killer (NK) cells. Alternatively, or additionally, ADCC activity of the molecule of interest may be assessed *in vivo*, e.g., in an animal model such as that disclosed in Clynes et al, PNAS USA 95:652-656 (1998).

A "T cell dysfunctional disorder" or a "T cell dysfunctional disease" is a disorder or condition of T-cells characterized by decreased responsiveness to antigenic stimulation. In a particular embodiment, a T-cell dysfunctional disorder is a disorder that is specifically associated with inappropriate increased signalling through PD-1. In another embodiment, T -cell dysfunctional disorder is one in which T-cells are anergic or have decreased ability to secrete cytokines, proliferate, or execute cytolytic activity. In a specific aspect, the decreased responsiveness results in ineffective control of a pathogen or tumor expressing an immunogen. Examples of T cell dysfunctional disorders characterized by T-cell dysfunction include unresolved acute infection, chronic infection and tumor immunity.

Percent (%) sequence identity" and "homology" with respect to a peptide, polypeptide or antibody sequence are defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2 or ALIGN software. Those skilled in the art can determine appropriate parameters for measuring alignment, including any algorithms needed to achieve maximal alignment over the full length of the sequences being compared.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

### Bispecific Binding Protein

The present invention relates to a bispecific binding protein binding to MUC-1 (e.g. represented by SEQ ID NO: 21) and CD40 (e.g. represented by SEQ ID NO. 22). Preferably, said MUC-1 is human MUC-1. Preferably, said CD40 is human CD40. More preferably, said MUC-1 is human MUC-1 and said CD40 is human CD40. In some embodiments, the bispecific binding protein comprises at least one binding site for MUC-1 and at least one binding site for CD40. In another embodiment, the bispecific binding protein comprises two binding sites for MUC-1 and two binding sites for CD40. In a further embodiment, the bispecific binding protein comprises at least two binding sites for MUC-1 and at least two binding sites for CD40. In a further embodiment, the bispecific binding protein comprises at least one binding site for MUC-1 and at least two binding sites for CD40. In a further embodiment, the bispecific binding protein comprises at least two binding sites for MUC-1 and at least one binding site for CD40.

In some embodiments, the two binding sites or the at least two binding sites for MUC-1 are identical to each other. In some embodiments, the two binding sites or the at least two binding sites for MUC-1 exhibit identical amino acid sequences. In another embodiment, the two binding sites or the at least two binding sites for MUC-1 are not identical to each other. In a further embodiment, the two binding sites or the at least two binding sites for MUC-1 have different amino acid sequences. In some embodiments, all binding sites for MUC-1 comprised in said bispecific binding protein are identical.

In some embodiments, the two binding sites or the at least two binding sites for CD40 are identical to each other. In some embodiments, the two binding sites or the at least two binding sites for CD40 exhibit identical amino acid sequences. In another embodiment, the two binding sites or the at least two binding sites for CD40 are not identical to each other. In a further embodiment, the two binding sites or the at least two binding sites for CD40 have different amino acid sequences. In some embodiments, all binding sites for CD40 comprised in said bispecific binding protein are identical.

In some embodiments, the bispecific binding protein comprises a first antigen binding moiety binding to MUC-1 and a second antigen binding moiety binding to CD40. The first and the second antigen binding moieties may comprise one or more polypeptide chains. In some embodiments, the first antigen binding moiety and the second antigen binding moiety are covalently linked. In a preferred embodiment, the first antigen binding moiety and the second antigen binding moiety are covalently linked in a fusion protein.

In some embodiments, the bispecific binding protein is a bispecific antibody. In some embodiments, the bispecific antibody comprises a first heavy chain and a first light chain forming a binding site binding to MUC-1 and a second heavy chain and a second light chain forming a binding site binding to CD40. In another embodiment, the bispecific antibody comprises a first antibody or antibody fragment binding to MUC-1 and a second antibody or antibody fragment binding to CD40. The first antibody or antibody fragment and the second antibody or antibody fragment may be covalently linked to each other. In a preferred embodiment, the first antibody or antibody fragment and the second antibody or antibody fragment are covalently linked to each other in a fusion protein.

In some embodiments, the bispecific binding protein comprises an anti-MUC-1 antibody. Preferably, the anti-MUC-1 antibody is a full-length antibody. The anti-MUC-1 antibody may be selected from an IgG, IgM, IgA, IgE and IgD antibody. Preferably, the anti-MUC-1 antibody is an IgG antibody. Further, the antibody may be selected from IgG1, IgG2, IgG3 and IgG4. Preferably, the anti-MUC-1 antibody is an IgG1 antibody. In another embodiment, the bispecific binding protein comprises an anti-MUC-1 antibody fragment. In some embodiments, the anti-MUC-1 antibody fragment binds MUC-1. In further embodiments, the anti-MUC-1 antibody fragment binds to the same antigen as a corresponding full-length anti-MUC-1 antibody. In some embodiments, the anti-MUC-1 antibody fragment binds MUC-1 with a KD that differs by not more than 10% from the KD with which the MUC-1 antibody comprised in FP1 binds MUC-1. The anti-MUC-1 antibody fragment may be a Fab fragment, a F(ab')₂ fragment, an Fv fragment or an scFv fragment.

In some embodiments, the bispecific binding protein comprises an anti-CD40 antibody. Preferably, the anti-CD40 antibody is a VHH antibody or a nanobody. More preferably, the anti-CD40 antibody is a VHH antibody. The anti-CD40 VHH antibody may be a camelid antibody. Further, the anti-CD40 VHH antibody may be humanized. In some embodiments, the bispecific binding protein comprises an anti-CD40 antibody fragment of said anti-CD40 VHH antibody. In some embodiments, the anti-CD40 antibody fragment binds CD40. In further embodiments, the anti-CD40 antibody fragment binds to the same antigen as a corresponding full-length anti-CD40 antibody. In some embodiments, the anti-CD40 antibody fragment binds CD40 with a KD that differs by not more than 10% from the KD with which the anti-CD40 VHH comprised in FP1 binds CD40. In another embodiment, the bispecific binding protein comprises an anti-CD40 antibody selected from an IgG, IgM, IgA, IgE and IgD antibody, preferably an IgG antibody, more preferably an IgG1 antibody.

In a preferred embodiment, the bispecific binding protein comprises an anti-MUC-1 IgG antibody and an anti-CD40 VHH antibody. In a further preferred embodiment, the bispecific binding protein comprises an anti-MUC-1 IgG antibody and an anti-CD40 VHH antibody fused to the C terminus of one of the heavy chains of the anti-MUC-1 IgG antibody. In a more preferred embodiment, the bispecific binding protein comprises an anti-MUC-1 IgG antibody and one anti-CD40 VHH antibody fused to the C terminus of each of the heavy chains of the anti-MUC-1 IgG antibody, respectively. Accordingly, the bispecific binding protein comprises one anti-MUC-1 IgG antibody and two anti-CD40 VHH antibodies (see Figure 14).

In one embodiment, the anti-MUC-1 antibody or the anti-MUC-1 antibody fragment comprises a VH CDR1 amino acid sequence according SEQ ID NO: 1, a VH CDR2 amino acid sequence according to SEQ ID NO: 2 and a VH CDR3 amino acid sequence according to SEQ ID NO: 3; and a VL CDR1 amino acid sequence according SEQ ID NO: 6, a VL CDR2 amino acid sequence according to SEQ ID NO: 7 and a VL CDR3 amino acid sequence according to SEQ ID NO: 8. In some embodiments, the anti-MUC-1 antibody or the anti-MUC-1 antibody fragment comprises a variable heavy chain amino acid sequence having at least 80%, at least 90%, at least 95% or at least 99% sequence identity with SEQ ID NO: 4 and/or a variable light chain amino acid sequence having at least 80%, at least 90%, at least 95% or at least 99% sequence identity with SEQ ID NO: 9. In a further embodiment, the anti-MUC-1 antibody or the anti-MUC-1 antibody fragment comprises a variable heavy chain amino acid sequence having at least 80% sequence identity with SEQ ID NO: 4 and a variable light chain amino acid sequence having at least 80% sequence identity with SEQ ID NO: 9. In a further embodiment, the anti-MUC-1 antibody or the anti-MUC-1 antibody fragment comprises a variable heavy chain amino acid sequence having at least 90% sequence identity with SEQ ID NO: 4 and a variable light chain amino acid sequence having at least 90% sequence identity with SEQ ID NO: 9. In a further embodiment, the anti-MUC-1 antibody or the anti-MUC-1 antibody fragment comprises a variable heavy chain amino acid sequence having at least 95% sequence identity with SEQ ID NO: 4 and a variable light chain amino acid sequence having at least 95% sequence identity with SEQ ID NO: 9. In a further embodiment, the anti-MUC-1 antibody or the anti-MUC-1 antibody fragment comprises a variable heavy chain amino acid sequence having at least 99% sequence identity with SEQ ID NO: 4 and a variable light chain amino acid sequence having at least 99% sequence identity with SEQ ID NO: 9. In some embodiments, the anti-MUC-1 antibody or the anti-MUC-1 antibody fragment binds to MUC-1 with a KD of 5 µM or less, preferably with a KD of less than 1 µM, most preferably with a KD in the nM range to pM range. In some embodiments, the anti-MUC-1 antibody or the anti-MUC-1 antibody fragment binds to MUC-1 with a KD ≤ 1 µM and ≥ 1 pM. In some embodiments, the anti-MUC-1 antibody exhibits at least 80%, at least 90% or at least 95% of the binding activity of an anti-MUC-1 antibody having a variable heavy chain amino acid sequence of SEQ ID NO: 4 and a variable light chain amino acid sequence of SEQ ID NO: 9. As the skilled person understands, if the present disclosure refers to the binding activity of a MUC-1 antibody, this refers to the binding activity of the MUC-1 antibody to MUC-1. The binding activity may be determined by KD measurement. In some embodiments, the anti-MUC-1 antibody comprises a heavy chain amino acid sequence having at least 80%, at least 90%, at least 95% or at least 99% sequence identity with SEQ ID NO: 5 and/or a light chain amino acid sequence having at least 80%, at least 90%, at least 95% or at least 99% sequence identity with SEQ ID NO: 10. In a further embodiment, the anti-MUC-1 antibody comprises a heavy chain amino acid sequence having at least 80% sequence identity with SEQ ID NO: 5 and a light chain amino acid sequence having at least 80% sequence identity with SEQ ID NO: 10. In a further embodiment, the anti-MUC-1 antibody comprises a heavy chain amino acid sequence having at least 90% sequence identity with SEQ ID NO: 5 and a light chain amino acid sequence having at least 90% sequence identity with SEQ ID NO: 10. In a further embodiment, the anti-MUC-1 antibody comprises a heavy chain amino acid sequence having at least 95% sequence identity with SEQ ID NO: 5 and a light chain amino acid sequence having at least 95% sequence identity with SEQ ID NO: 10. In a further embodiment, the anti-MUC-1 antibody comprises a heavy chain amino acid sequence having at least 99% sequence identity with SEQ ID NO: 5 and a light chain amino acid sequence having at least 99% sequence identity with SEQ ID NO: 10. In some embodiments, the anti-MUC-1 antibody exhibits at least 80%, at least 90% or at least 95% of the binding activity of an anti-MUC-1 antibody having a heavy chain amino acid sequence of SEQ ID NO: 5 and a light chain amino acid sequence of SEQ ID NO: 10. The binding activity may be determined by KD measurement.

In some embodiments, the anti-CD40 VHH (or the anti-CD40 antibody fragment) comprises a CDR1 amino acid sequence according to SEQ ID NO: 12, a CDR2 amino acid sequence according to SEQ ID NO: 13 and a CDR3 amino acid sequence according to SEQ ID NO: 14. In some embodiments, the anti-CD40 VHH (or the anti-CD40 antibody fragment) comprises a CDR1 amino acid sequence according to SEQ ID NO: 12 or a variant thereof, a CDR2 amino acid sequence according to SEQ ID NO: 13 or a variant thereof and a CDR3 amino acid sequence according to SEQ ID NO: 14 or a variant thereof, wherein each CDR variant comprises at most two amino acid exchanges. In some embodiments, the anti-CD40 VHH (or the anti-CD40 antibody fragment) comprises a CDR1 amino acid sequence according to SEQ ID NO: 12 or a variant thereof, a CDR2 amino acid sequence according to SEQ ID NO: 13 or a variant thereof and a CDR3 amino acid sequence according to SEQ ID NO: 14 or a variant thereof, wherein each CDR variant comprises at most one amino acid exchange. In some embodiments, the anti-CD40 VHH or the anti-CD40 antibody fragment binds to CD40 with a KD of < 1 µM, preferably with a KD of < 100 nM. In some embodiments, the anti-CD40 VHH (or anti-CD40 antibody fragment) comprising a CDR1 variant, a CDR2 variant and/or a CDR3 variant exhibits at least 80%, at least 90% or at least 95% of the binding activity of an anti-CD40 VHH with an amino acid sequence of SEQ ID NO:15. As the skilled person understands, if the present disclosure refers to the binding activity of an anti-CD40 VHH, this refers to the binding activity of the anti-CD40 VHH to CD40. The binding activity may be determined by KD measurement. In some embodiments, the anti-CD40 VHH (or anti-CD40 antibody fragment) comprises a CDR3 amino acid sequence according to SEQ ID NO: 14. In some embodiments, the anti-CD40 VHH (or the anti-CD40 antibody fragment) comprises a CDR3 amino acid sequence according to SEQ ID NO: 14 and exhibits at least 80%, at least 90% or at least 95% of the binding activity of an anti-CD40 VHH with an amino acid sequence of SEQ ID NO:15. The binding activity may be determined by KD measurement. In a further embodiment, the anti-CD40 VHH (or the anti-CD40 antibody fragment) comprises an amino acid sequence having at least 60%, at least 70%, at least 80%, at least 90%, at least 95% or at least 99% sequence identity with SEQ ID NO: 15. In a further embodiment, the anti-CD40 VHH (or the anti-CD40 antibody fragment) comprises an amino acid sequence having at least 60%, at least 70%, at least 80%, at least 90%, at least 95% or at least 99% sequence identity with SEQ ID NO: 15 and exhibits at least 80%, at least 90% or at least 95% of the binding activity of an anti-CD40 VHH with an amino acid sequence of SEQ ID NO:15. The binding activity may be determined by KD measurement.

In some embodiments, the anti-MUC-1 antibody or the anti-MUC-1 antibody fragment and the anti-CD40 VHH (or the anti-CD40 antibody fragment) are covalently linked to each other. In a further embodiment, the anti-MUC-1 antibody or the anti-MUC-1 antibody fragment and the anti-CD40 VHH (or the anti-CD40 antibody fragment) are covalently linked with or without an amino acid linker sequence in a fusion protein. In a preferred embodiment, the anti-MUC-1 antibody or the anti-MUC-1 antibody fragment and the anti-CD40 VHH (or the anti-CD40 antibody fragment) are covalently linked with an amino acid linker sequence in a fusion protein. The anti-MUC-1 antibody or the anti-MUC-1 antibody fragment may be linked to two anti-CD40 VHH antibodies (or anti-CD40 antibody fragments), wherein each anti-CD40 VHH antibody (or anti-CD40 antibody fragment) is linked via a linker amino acid sequence to the anti-MUC-1 antibody or the anti-MUC-1 antibody fragment. In some embodiments, the bispecific binding protein comprises an anti-MUC-1 antibody or an anti-MUC-1 antibody fragment, two anti-CD40 VHH antibodies (or anti-CD40 antibody fragments) and two linker sequences. Preferably, the amino acid linker sequence consists of amino acids selected from G and S. Further, the linker may exhibit a length of 10-30 amino acids. In some embodiments, the amino acid linker sequence consists of amino acids selected from G and S and exhibits a length of 10-30 amino acids. More preferably, the amino acid linker sequence comprises an amino acid sequence according to SEQ ID NO: 11 The amino acid linker sequence may consist of an amino acid sequence according to SEQ ID NO:11.

In some embodiments, the bispecific binding protein comprises a light chain amino acid sequence having at least 80%, at least 90%, at least 95% or at least 99% sequence identity with SEQ ID NO: 10 and/or a heavy chain amino acid sequence having at least 80%, at least 90%, at least 95% or at least 99% sequence identity with SEQ ID NO: 16. In some embodiments, the bispecific binding protein comprises a light chain amino acid sequence having at least 80% sequence identity with SEQ ID NO: 10 and a heavy chain amino acid sequence having at least 80% sequence identity with SEQ ID NO: 16. In some embodiments, the bispecific binding protein comprises a light chain amino acid sequence having at least 90% sequence identity with SEQ ID NO: 10 and a heavy chain amino acid sequence having at least 90% sequence identity with SEQ ID NO: 16. In some embodiments, the bispecific binding protein comprises a light chain amino acid sequence having at least 95% sequence identity with SEQ ID NO: 10 and a heavy chain amino acid sequence having at least 95% sequence identity with SEQ ID NO: 16. In some embodiments, the bispecific binding protein comprises a light chain amino acid sequence having at least 99% sequence identity with SEQ ID NO: 10 and a heavy chain amino acid sequence having at least 99% sequence identity with SEQ ID NO: 16.

In a preferred embodiment, the bispecific binding protein comprises a light chain amino acid sequence according to SEQ ID NO: 10 and a heavy chain amino acid sequence according to SEQ ID NO: 16. In a further preferred embodiment, the bispecific binding protein comprises two light chain amino acid sequences according to SEQ ID NO: 10 and two heavy chain amino acid sequences according to SEQ ID NO: 16. In yet a further preferred embodiment, the bispecific binding protein comprises two light chain amino acid sequences according to SEQ ID NO: 10 and two heavy chain amino acid sequences according to SEQ ID NO: 16, wherein the two heavy chain amino acid sequences are linked via a disulphide bridge. In yet a further preferred embodiment, the bispecific binding protein comprises two light chain amino acid sequences according to SEQ ID NO: 10 and two heavy chain amino acid sequences according to SEQ ID NO: 16, wherein the two heavy chain amino acid sequences are linked via two disulphide bridges. The two heavy chains and the two light chains may assemble into an anti-MUC-1 antibody, wherein an anti-CD40 VHH antibody is present at the C-terminus of each heavy chain of the anti-MUC-1 antibody (see Figure 14).

In another preferred embodiment, the bispecific binding protein comprises two anti-human CD40 VHH domains (SEQ ID NO: 15) covalently linked via a flexible 15aa GS-linkers (SEQ ID NO: 11) to the C-terminus of the heavy chains of a fully human anti-MUC-1 IgG1 antibody (heavy chain VH: SEQ ID NO: 4; light chain VL: SEQ ID NO: 9), wherein the first anti-CD40 VHH is linked via GS-linker to the C-terminus of the first heavy chain of the anti-MUC-1 IgG1 antibody and wherein the second anti-CD40 VHH is linked via GS-linkerto the C-terminus of the second heavy chain of the anti-MUC-1 IgG1 antibody. In another preferred embodiment, the bispecific binding protein comprises two anti-human CD40 VHH domains (SEQ ID NO: 15) covalently linked via a flexible 15aa GS-linkers (SEQ ID NO: 11) to the C-terminus of the heavy chains of a fully human anti-MUC-1 IgG1 antibody (heavy chain: SEQ ID NO: 5; light chain: SEQ ID NO: 10), wherein the first anti-CD40 VHH is linked via GS-linker to the C-terminus of the first heavy chain of the anti-MUC-1 IgG1 antibody and wherein the second anti-CD40 VHH is linked via GS-linker to the C-terminus of the second heavy chain of the anti-MUC-1 IgG1 antibody. The bispecific binding protein can further be described as IgG1 fusion protein with two heavy chains according to SEQ ID NO: 16 associated with two light chains according to SEQ ID NO: 10, wherein, preferably, the two heavy chains and are linked by two IgG1 interchain disulfide bonds. The format of the molecule enables conditional activation of CD40 on antigen-presenting cells to kick-start T-cell response.

**Table 1 - Sequences**

| **SEQ ID NO** | **Description** | **Amino acid sequence** |
|---|---|---|
| SEQ ID NO: 1 | VH CDR1 (anti MUC-1) | GYSFTGHY |
| SEQ ID NO: 2 | VH CDR2 (anti MUC-1) | IDPVTGGT |
| SEQ ID NO: 3 | VH CDR3 (anti MUC-1) | AREVTGDRGQFDK |
| SEQ ID NO: 4 | variable heavy chain VH (anti MUC-1) | EVQLVQSGAEVKKPGASVKVSCKASGYSFTGHYMHWVRQAPGQGLEWMGWIDPVTGGT KYAQKFQGWVTMTRDTSISTAYMELSRLRSDDTAVYYCAREVTGDRGQFDKWGQGTLV TVSS |
| SEQ ID NO: 5 | heavy chain (anti MUC-1 IgG1) | EVQLVQSGAEVKKPGASVKVSCKASGYSFTGHYMHWVRQAPGQGLEWMGWIDPVTGGT KYAQKFQGWVTMTRDTSISTAYMELSRLRSDDTAVYYCAREVTGDRGQFDKWGQGTLV TVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCDKTHTCPPC PAPEAAGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPRE PQVYTLPPSREEMTKNQVSLTCLVKGFYPSDIAVEWESNGQPENNYKTTPPVLDSDGS FFLYSKLTVDKSRWQQGNVFSCSVMHEALHNHYTQKSLSLSPG |
| SEQ ID NO: 6 | VL CDR1 (anti MUC-1) | NIGSKS |
| SEQ ID NO: 7 | VL CDR2 (anti MUC-1) | YGS |
| SEQ ID NO: 8 | VL CDR3 (anti MUC-1) | QVWDSSSDWV |
| SEQ ID NO: 9 | variable light chain VL (anti MUC-1) | SYVLTQPPSVSVAPGKTARITCGGNNIGSKSVHWYQQKPGQAPVLVIYYGSNRPSGIP ERFSGSNSGNTATLTISRVEAGDEADYYCQVWDSSSDWVFGGGTKLTVL |
| SEQ ID NO: 10 | light chain (anti MUC-1) | SYVLTQPPSVSVAPGKTARITCGGNNIGSKSVHWYQQKPGQAPVLVIYYGSNRPSGIP ERFSGSNSGNTATLTISRVEAGDEADYYCQVWDSSSDWVFGGGTKLTVLGQPKAAPSV TLFPPSSEELQANKATLVCLISDFYPGAVTVAWKADSSPVKAGVETTTPSKQSNNKYA ASSYLSLTPEQWKSHKSYSCQVTHEGSTVEKTVAPTECS |
| SEQ ID NO: 11 | linker sequence | GSGGGSGGSGGGGSG |
| SEQ ID NO: 12 | VHH CDR1 (anti CD40) | GFTFSSAT |
| SEQ ID NO: 13 | VHH CDR2 (anti CD40) | ISSVGGGT |
| SEQ ID NO: 14 | VHH CDR3 (anti CD40) | VTMRSIRNPDFGSR |
| SEQ ID NO: 15 | VHH (anti CD40) | EVQLVESGGGVVQPGGSLRLSCAASGFTFSSATMGWFRQAPGKGLEWVAYISSVGGGT DYADSVKGRFTISRDNAKNTLYLQMNSLRAEDTAVYYCVTMRSIRNPDFGSRGQGTLV TVSSA |
| SEQ ID NO: 16 | heavy chain-linker-VHH (anti MUC-1 × CD40) | EVQLVQSGAEVKKPGASVKVSCKASGYSFTGHYMHWVRQAPGQGLEWMGWIDPVTGGT KYAQKFQGWVTMTRDTSISTAYMELSRLRSDDTAVYYCAREVTGDRGQFDKWGQGTLV TVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCDKTHTCPPC PAPEAAGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPRE PQVYTLPPSREEMTKNQVSLTCLVKGFYPSDIAVEWESNGQPENNYKTTPPVLDSDGS FFLYSKLTVDKSRWQQGNVFSCSVMHEALHNHYTQKSLSLSPGGSGGGSGGSGGGGSG EVQLVESGGGVVQPGGSLRLSCAASGFTFSSATMGWFRQAPGKGLEWVAYISSVGGGT DYADSVKGRFTISRDNAKNTLYLQMNSLRAEDTAVYYCVTMRSIRNPDFGSRGQGTLV TVSSA |
| SEQ ID NO: 17 | anti-MUC1-anti-CD40-VHH heavy chain (nucleotide) | ATGGAAACAGACACCCTGCTGCTGTGGGTGCTGCTGCTGTGGGTGCCCGGCTCCACAG GCGAGGTGCAGCTGGTGCAGTCTGGGGCCGAGGTGAAGAAACCAGGCGCCAGCGTGAA GGTGTCCTGCAAGGCCAGCGGCTACAGCTTCACCGGCCACTACATGCACTGGGTGCGC CAGGCTCCAGGCCAGGGACTGGAATGGATGGGCTGGATCGACCCTGTGACCGGCGGCA CCAAATACGCCCAGAAGTTCCAGGGCTGGGTCACCATGACCCGGGACACCAGCATCAG CACCGCCTACATGGAACTGAGCCGGCTGAGAAGCGACGACACCGCCGTGTACTACTGC GCCAGAGAAGTGACAGGCGACCGGGGCCAGTTCGATAAGTGGGGCCAGGGCACACTCG TGACCGTGAGCAGCGCCTCCACCAAGGGCCCATCGGTCTTCCCCCTGGCACCCTCCTC CAAGAGCACCTCTGGGGGCACAGCGGCCCTGGGCTGCCTGGTCAAGGACTACTTCCCC GAACCGGTGACGGTGTCGTGGAACTCAGGCGCCCTGACCAGCGGCGTGCACACCTTCC CGGCTGTCCTACAGTCCTCAGGACTCTACTCCCTCAGCAGCGTGGTGACCGTGCCCTC |
| | | CAGCAGCTTGGGCACCCAGACCTACATCTGCAACGTGAATCACAAGCCCAGCAACACC AAGGTGGACAAGAGAGTTGAACCCAAGAGCTGCGACAAGACCCACACCTGTCCTCCAT GTCCTGCTCCTGAAGCTGCCGGGGGCCCTTCCGTGTTTCTGTTCCCTCCAAAGCCTAA GGACACCCTGATGATCAGCAGAACCCCTGAAGTGACCTGCGTGGTGGTGGATGTGTCT CACGAGGACCCCGAAGTGAAGTTCAATTGGTACGTGGACGGCGTGGAAGTTCACAACG CAAAGACTAAACCTCGAGAAGAGCAGTATAATTCTACATATCGTGTCGTGTCCGTTCT CACCGTGCTCCACCAGGATTGGTTGAATGGCAAAGAGTATAAGTGCAAGGTGTCCAAC AAAGCACTTCCAGCACCCATCGAAAAGACCATTTCTAAGGCCAAGGGGCAACCCCGAG AACCTCAAGTGTATACTCTGCCCCCTTCTCGCGAAGAGATGACTAAAAACCAGGTCAG TCTTACTTGCTTGGTCAAAGGCTTCTACCCTAGCGACATCGCCGTCGAATGGGAAAGC AATGGGCAACCAGAAAATAACTACAAGACAACCCCACCTGTCCTCGATTCAGACGGCA GTTTTTTTCTGTATAGTAAACTGACAGTGGACAAAAGCCGCTGGCAGCAGGGTAACGT CTTTTCATGTTCAGTTATGCATGAGGCACTTCACAATCATTACACCCAAAAGAGCCTG TCTCTCAGTCCAGGTGGTAGTGGTGGCGGGTCTGGAGGCTCAGGCGGGGGTGGTTCAG GGGAAGTACAGCTCGTAGAATCAGGAGGAGGGGTGGTCCAACCAGGCGGCTCACTGCG ACTGTCATGCGCAGCTTCAGGATTCACATTCAGCTCTGCAACCATGGGCTGGTTTAGG CAAGCCCCCGGAAAGGGGCTCGAATGGGTCGCATACATTTCATCCGTAGGGGGGGGTA CTGATTACGCAGACTCAGTAAAGGGACGTTTTACAATATCTCGCGACAATGCAAAAAA TACACTGTATCTTCAAATGAACTCACTCAGGGCTGAGGATACCGCCGTATATTACTGT GTCACAATGCGGTCAATACGTAACCCAGACTTTGGGTCACGGGGGCAAGGGACACTCG TGACAGTTTCATCTGCATAATGA |
| SEQ ID NO:18 | anti-MUC-1 lambda light chain (nucleotide) | ATGCGGGCTCTCCTGGCCAGGCTGCTGCTGTGCGTGCTGGTGGTGAGCGACAGCAAGG GCAGCTACGTGCTGACTCAGCCTCCTAGCGTCAGCGTGGCTCCCGGCAAAACTGCTAG GATCACATGTGGCGGCAACAACATCGGCAGCAAGAGCGTGCACTGGTATCAGCAGAAG CCCGGCCAAGCCCCAGTGCTCGTGATCTACTACGGCAGCAATAGGCCTTCCGGCATCC CAGAGAGATTCTCCGGCAGCAACTCCGGCAATACTGCCACACTGACAATCTCTAGGGT GGAGGCCGGAGACGAGGCTGACTACTATTGCCAAGTGTGGGACAGCAGCTCCGACTGG GTGTTCGGCGGAGGCACTAAGCTGACTGTGCTGGGACAGCCTAAGGCCGCTCCTAGCG TGACTCTGTTCCCACCAAGCAGCGAGGAGCTGCAAGCCAATAAAGCCACTCTGGTGTG TCTGATCAGCGATTTCTACCCCGGCGCCGTGACAGTCGCTTGGAAGGCTGATTCCAGC CCAGTGAAGGCCGGCGTGGAGACTACAACTCCTTCCAAGCAGTCCAACAACAAGTACG CCGCCAGCTCCTATCTGTCTCTGACTCCAGAGCAGTGGAAGAGCCACAAGAGCTACAG CTGCCAAGTGACTCACGAGGGCTCCACTGTGGAGAAGACAGTGGCCCCTACAGAGTGC AGCTGATAG |
| SEQ ID NO:19 | anti-MUC-1-anti-CD40-VHH heavy chain | METDTLLLWVLLLWVPGSTGEVQLVQSGAEVKKPGASVKVSCKASGYSFTGHYMHWVR QAPGQGLEWMGWIDPVTGGTKYAQKFQGWVTMTRDTSISTAYMELSRLRSDDTAVYYC AREVTGDRGQFDKWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNVNHKPSNT KVDKRVEPKSCDKTHTCPPCPAPEAAGGPSVFLFPPKPKDTLMISRTPEVTCVVVDVS HEDPEVKFNWYVDGVEVHNAKTKPREEQYNSTYRVVSVLTVLHQDWLNGKEYKCKVSN KALPAPIEKTISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEALHNHYTQKSL SLSPGGSGGGSGGSGGGGSGEVQLVESGGGVVQPGGSLRLSCAASGFTFSSATMGWFR QAPGKGLEWVAYISSVGGGTDYADSVKGRFTISRDNAKNTLYLQMNSLRAEDTAVYYC VTMRSIRNPDFGSRGQGTLVTVSSA |
| SEQ ID NO:20 | anti-MUC-1 light chain | MRALLARLLLCVLVVSDSKGSYVLTQPPSVSVAPGKTARITCGGNNIGSKSVHWYQQK PGQAPVLVIYYGSNRPSGIPERFSGSNSGNTATLTISRVEAGDEADYYCQVWDSSSDW VFGGGTKLTVLGQPKAAPSVTLFPPSSEELQANKATLVCLISDFYPGAVTVAWKADSS PVKAGVETTTPSKQSNNKYAASSYLSLTPEQWKSHKSYSCQVTHEGSTVEKTVAPTEC S |
| SEQ ID NO: 21 | human MUC-1 amino acid sequence (https://www.u niprot.org/uni protkb/P15941 /entry#sequences (P15941-1)) | MTPGTQSPFFLLLLLTVLTVVTGSGHASSTPGGEKETSATQRSSVPSSTEKNAVSMTS SVLSSHSPGSGSSTTQGQDVTLAPATEPASGSAATWGQDVTSVPVTRPALGSTTPPAH DVTSAPDNKPAPGSTAPPAHGVTSAPDTRPAPGSTAPPAHGVTSAPDTRPAPGSTAPP AHGVTSAPDTRPAPGSTAPPAHGVTSAPDTRPAPGSTAPPAHGVTSAPDTRPAPGSTA PPAHGVTSAPDTRPAPGSTAPPAHGVTSAPDTRPAPGSTAPPAHGVTSAPDTRPAPGS TAPPAHGVTSAPDTRPAPGSTAPPAHGVTSAPDTRPAPGSTAPPAHGVTSAPDTRPAP GSTAPPAHGVTSAPDTRPAPGSTAPPAHGVTSAPDTRPAPGSTAPPAHGVTSAPDTRP APGSTAPPAHGVTSAPDTRPAPGSTAPPAHGVTSAPDTRPAPGSTAPPAHGVTSAPDT RPAPGSTAPPAHGVTSAPDTRPAPGSTAPPAHGVTSAPDTRPAPGSTAPPAHGVTSAP DTRPAPGSTAPPAHGVTSAPDTRPAPGSTAPPAHGVTSAPDTRPAPGSTAPPAHGVTS APDTRPAPGSTAPPAHGVTSAPDTRPAPGSTAPPAHGVTSAPDTRPAPGSTAPPAHGV TSAPDTRPAPGSTAPPAHGVTSAPDTRPAPGSTAPPAHGVTSAPDTRPAPGSTAPPAH GVTSAPDTRPAPGSTAPPAHGVTSAPDTRPAPGSTAPPAHGVTSAPDTRPAPGSTAPP AHGVTSAPDTRPAPGSTAPPAHGVTSAPDTRPAPGSTAPPAHGVTSAPDTRPAPGSTA PPAHGVTSAPDTRPAPGSTAPPAHGVTSAPDTRPAPGSTAPPAHGVTSAPDTRPAPGS TAPPAHGVTSAPDTRPAPGSTAPPAHGVTSAPDTRPAPGSTAPPAHGVTSAPDTRPAP GSTAPPAHGVTSAPDNRPALGSTAPPVHNVTSASGSASGSASTLVHNGTSARATTTPA SKSTPFSIPSHHSDTPTTLASHSTKTDASSTHHSSVPPLTSSNHSTSPQLSTGVSFFF LSFHISNLQFNSSLEDPSTDYYQELQRDISEMFLQIYKQGGFLGLSNIKFRPGSVVVQ LTLAFREGTINVHDVETQFNQYKTEAASRYNLTISDVSVSDVPFPFSAQSGAGVPGWG IALLVLVCVLVALAIVYLIALAVCQCRRKNYGQLDIFPARDTYHPMSEYPTYHTHGRY VPPSSTDRSPYEKVSAGNGGSSLSYTNPAVAATSANL |
| SEQ ID NO: 22 | human CD40 amino acid sequence (https://www.u niprot.org/uni protkb/P25942 /entry#sequences (P25942-1)) | MVRLPLQCVLWGCLLTAVHPEPPTACREKQYLINSQCCSLCQPGQKLVSDCTEFTETE CLPCGESEFLDTWNRETHCHQHKYCDPNLGLRVQQKGTSETDTICTCEEGWHCTSEAC ESCVLHRSCSPGFGVKQIATGVSDTICEPCPVGFFSNVSSAFEKCHPWTSCETKDLVV QQAGTNKTDVVCGPQDRLRALVVIPIIFGILFAILLVLVFIKKVAKKPTNKAPHPKQE PQEINFPDDLPGSNTAAPVQETLHGCQPVTQEDGKESRISVQERQ |
| SEQ ID NO: 23 | MUC-1 epitope sequence bound by anti MUC-1 antibody | APDTRPAPGSTAPPA |

In case of discrepancies between the information in Table 1 above and the information in the enclosed sequence listing file, the information in Table 1 above shall prevail.

In some embodiments, the bispecific binding protein comprises an anti-MUC-1 antibody or an anti-MUC-1 antibody fragment comprising a VH CDR1 amino acid sequence according SEQ ID NO: 1, a VH CDR2 amino acid sequence according to SEQ ID NO: 2 and a VH CDR3 amino acid sequence according to SEQ ID NO: 3; and a VL CDR1 amino acid sequence according SEQ ID NO: 6, a VL CDR2 amino acid sequence according to SEQ ID NO: 7 and a VL CDR3 amino acid sequence according to SEQ ID NO: 8 and an anti-CD40 VHH. The anti-MUC-1 antibody or the anti-MUC-1 antibody fragment and the anti-CD40 VHH may be covalently linked in a fusion protein. In some embodiments, the anti-CD40 VHH is linked to the C-terminus of the heavy chain or light chain of the anti-MUC-1 antibody or the anti-MUC-1 antibody fragment.

In some embodiments, the bispecific binding protein comprises an anti-MUC-1 antibody or the anti-MUC-1 antibody fragment comprising a variable heavy chain amino acid sequence according to SEQ ID NO: 4 and a variable light chain amino acid sequence according to SEQ ID NO: 9 and an anti-CD40 VHH. The anti-MUC-1 antibody or the anti-MUC-1 antibody fragment and the anti-CD40 VHH may be covalently linked in a fusion protein. In some embodiments, the anti-CD40 VHH is linked to the C-terminus of the heavy chain or light chain of the anti-MUC-1 antibody or the anti-MUC-1 antibody fragment.

In some embodiments, the bispecific binding protein comprises an anti-MUC-1 antibody comprising a heavy chain amino acid sequence according to SEQ ID NO: 5 and a light chain amino acid sequence according to SEQ ID NO: 10 and an anti-CD40 VHH. The anti-MUC-1 antibody and the anti-CD40 VHH may be covalently linked in a fusion protein. In some embodiments, the anti-CD40 VHH is linked to the C-terminus of the heavy chain or light chain of the anti-MUC-1 antibody.

In some embodiments, the bispecific binding protein comprises an anti-CD40 VHH comprising a CDR3 amino acid sequence according to SEQ ID NO: 14 and an anti-MUC-1 antibody or an anti-MUC-1 antibody fragment. The anti-MUC-1 antibody or the anti-MUC-1 antibody fragment and the anti-CD40 VHH may be covalently linked in a fusion protein. In some embodiments, the anti-CD40 VHH is linked to the C-terminus of the heavy chain or light chain of the anti-MUC-1 antibody or the anti-MUC-1 antibody fragment.

In some embodiments, the bispecific binding protein comprises an anti-CD40 VHH comprising a CDR1 amino acid sequence according to SEQ ID NO: 12, a CDR2 amino acid sequence according to SEQ ID NO: 13 and a CDR3 amino acid sequence according to SEQ ID NO: 14 and an anti-MUC-1 antibody or an anti-MUC-1 antibody fragment. The anti-MUC-1 antibody or the anti-MUC-1 antibody fragment and the anti-CD40 VHH may be covalently linked in a fusion protein. In some embodiments, the anti-CD40 VHH is linked to the C-terminus of the heavy chain or light chain of the anti-MUC-1 antibody or the anti-MUC-1 antibody fragment.

In some embodiments, the bispecific binding protein comprises an anti-CD40 VHH comprising an amino acid sequence of SEQ ID NO:15 and an anti-MUC-1 antibody or an anti-MUC-1 antibody fragment. The anti-MUC-1 antibody or the anti-MUC-1 antibody fragment and the anti-CD40 VHH may be covalently linked in a fusion protein. In some embodiments, the anti-CD40 VHH is linked to the C-terminus of the heavy chain or light chain of the anti-MUC-1 antibody or the anti-MUC-1 antibody fragment.

In some embodiments, the bispecific binding protein binds to MUC-1 with a dissociation constant (KD) of < 1×10⁻⁶ M and/or the bispecific binding protein binds to CD40 with a dissociation constant (KD) of < 1×10⁻⁶ M.

In some embodiments, the bispecific binding protein comprises an anti-MUC-1 antibody or an anti-MUC-1 antibody fragment as defined herein and a further binding moiety. In some embodiments, the bispecific binding protein comprises anti-CD40 VHH as defined herein and a further binding moiety.

In some embodiments, the bispecific binding protein induces CD40 activation on B cells in the presence of MUC-1-expressing tumor cells. Conversely, the bispecific binding protein does not induce CD40 activation on B cells in the absence of MUC-1-expressing tumor cells or in the absence of MUC-1 antigen. CD40 activation in B cells may be measured by any assay or method, such as the methods described in Example 8. Preferably, the induction of CD40 activation by the bispecific binding protein is measured as follows: Isolated B cells are co-cultured in the presence or absence of tumor cells derived from a MUC-1-expressing tumor cell line (e.g., SNU-407, HPAC or T47D) at a 1:1 ratio in the presence of a bispecific binding protein as described herein. CD86 cell surface expression on the B cells cultured in the presence of a bispecific protein in the presence of tumor cells expressing MUC-1 is increased by at least by 50%, more preferably by 100% (2-fold; i.e. to 2-fold the CD86 cell surface expression on the B cells compared to the CD86 cell surface expression on the B cells in the control setup), more preferably 4-fold, more preferably 6-fold, more preferably 8-fold as compared to B cells cultured in the presence of a bispecific protein as described herein in the absence of tumor cells expressing MUC-1 or in the presence of MUC-1 negative tumor cells.

In some embodiments, the bispecific binding protein induces increased CD86 cell surface expression on B cells in the presence of MUC-1-expressing tumor cells, wherein increased CD86 cell surface expression is measured by culturing isolated B cells in the presence tumor cells derived from a MUC-1-expressing tumor cell line (e.g., SNU-407, HPAC or T47D, preferably T47D) in the presence of a bispecific binding protein as described herein, wherein the isolated B cells and the tumor cells derived from a MUC-1-expressing tumor cell line are present at a 1:1 ratio and the bispecific binding protein is present at a concentration between 1-10 nM and wherein the CD86 cell surface expression in the B cells is increased over B cells cultured under the same conditions but in absence of tumor cells derived from a MUC-1-expressing tumor cell line. CD86 cell surface expression on the B cells cultured in the presence of a bispecific protein in the presence of tumor cells expressing MUC-1 may be increased by at least by 50%, more preferably by 100% (i.e. 2-fold), more preferably 4-fold, more preferably 6-fold, more preferably 8-fold as compared to B cells cultured in the presence of a bispecific protein as described herein in the absence of tumor cells expressing MUC-1.

Specifically, the assay may be performed as follows: B cells are isolated from healthy blood donor PBMCs using magnetic bead-based separation of B cells from other immune cells using EasySep^{™} Human B Cell Isolation Cocktail, Stemcell Technologies, and manufacturers protocol. The bispecific binding protein and technical control anti-CD40 antibody selicrelumab or sotigalimab or non-binding isotype control antibody are adjusted to 120 nM concentration and titrations are carried out in serial dilution 1:5 or 1:10 using B cell medium (RPMI 1640 cell culture medium, 10% FBS, 2 mM L-Glutamine, 1% Penicillin/Streptomycin). The serial dilutions of bispecific binding protein are applied to B cell and tumor cell co-cultures and diluted 1:4. After 24 hrs of incubation, B cells are collected and prepared for flow cytometry analysis. B cells are blocked with Beriglobin at 4°C for 30 min, washed with PBS and stained at 4°C for 30 min with Live/Dead Fixable Near-IR Dead Cell Stain Kit (1:2000), Anti-CD19 fluorochrome 1 labeled antibody to detect B cells and anti-69 fluorochrome 2, anti-CD86 fluorochrome 3 labeled antibody to detect B cell activation. Measurement of stained cells is conducted with a frequently CST-calibrated flow cytometer. Anti-CD40 antibody selicrelumab or sotigalimab are used as a positive control. Non-binding isotype control is used as negative control. B cells without MUC-1 expressing tumor cells may also serve as negative control condition. MUC-1 negative tumor cells may also serve as negative controls.

In some embodiments, the bispecific binding protein induces CD40 activation on dendritic cells in the presence of MUC-1-expressing tumor cells. Conversely, the bispecific binding protein does not induce CD40 activation on dendritic cells in the absence of MUC-1-expressing tumor cells or in the absence of MUC-1 antigen. CD40 activation in dendritic cells may be measured by any assay or method. For example, isolated B cells are co-cultured in the presence or absence of tumor cells derived from a MUC-1-expressing tumor cell line (e.g., HCC-827) at a 1:1 ratio in the presence of a bispecific binding protein as described herein at a concentration between 1-10 nM. IL-12 expression is increased in dendritic cells cultured in the presence of a bispecific protein as described herein in the presence of tumor cells expressing MUC-1 by at least 50%, at least 60% or at least 70% as compared to dendritic cells cultured in the presence of a bispecific protein as described herein in the absence of tumor cells expressing MUC-1 or in the presence of MUC-1 negative tumor cells.

The assay may be performed as follows: Dendritic cells (DCs) are derived from monocytes. Monocytes are isolated from healthy blood donor PBMCs using magnetic bead-based separation of monocytes from other immune cells using EasySep^{™} Human Monocyte Enrichment Cocktail w/o CD16 Depletion, Stemcell Technologies, and manufacturers protocol. Isolated monocytes are differentiated in DCs medium (RPMI 1640 cell culture medium, 10% FBS, 2 mM L-Glutamine, 1% Penicillin/Streptomycin) and 50 ng/ml GM-CSF, 200 ng/ml IL-4 used for differentiation into dendritic cells for at least 5 days. Differentiated dendritic cells are collected and co-cultured in the presence or absence of tumor cells derived from a MUC-1-expressing tumour cell line (e.g. HCC827) in a 1:1 ratio and presence of bispecific binding protein or other treatment conditions. The bispecific binding protein and technical control anti-CD40 antibody selicrelumab or sotigalimab or non-binding isotype control antibody are adjusted to 120 nM concentration and titrations are carried out in serial dilution 1:5 or 1:10 using DC medium (RPMI 1640 cell culture medium, 10% FBS, 2 mM L-Glutamine, 1% Penicillin/Streptomycin). The serial dilutions of bispecific binding protein are applied to DCs and tumor cell co-cultures and diluted 1:4. After 24 hrs of incubation, DCs are collected for further analysis of costimulatory marker expression via flow cytometry as a metric of CD40 activation or supernatants are collected for IL-12 cytokine expression analysis using ELISA according to the manufacturers protocols. For flow cytometry analysis, DCs are blocked with Beriglobin at 4°C for 30 min, washed with PBS and stained at 4°C for 30 min with Live/Dead Fixable Near-IR Dead Cell Stain Kit (1:2000), Anti-CD45 fluorochrome 1 labeled antibody to detect DCs and anti-80 fluorochrome 2, anti-CD86 fluorochrome 3 labeled antibody to detect DCs activation. Measurement of stained cells is conducted with a frequently CST-calibrated flow cytometer.

In one embodiment, the bispecific binding protein does not induce CD40 activation on dendritic cells in the presence of MUC-1-negative cells. In some embodiments, the bispecific binding protein does not induce CD40 activation on dendritic cells in the presence of MUC-1-negative cancer cells.

In some embodiments, the bispecific binding protein specifically binds to CD40 and specifically binds to MUC-1. Binding of the bispecific binding protein to CD40 and/or to MUC-1 can be individually analyzed with any suitable assay (e.g. surface plasmon resonance (Biacore) or radiolabeled antigen binding (RIA) assay). In some embodiments, the bispecific binding protein binds to MUC1 with a dissociation constant (KD) of < 1×10⁻⁶M, < 1×10⁻⁷M, < 1×10⁻⁸M, < 1×10⁻⁹M, or < 1×10⁻¹⁰M. The KD may be measured by SPR (Biacore) using and immobilized human MUC-1 peptide (SEQ ID NO: 23: APDTRPAPGSTAPPA) (Running Buffer HBS-N, pH 7.4, 350 mM NaCl). In some embodiments, the bispecific binding protein binds to CD40 with a dissociation constant (KD) of < 1×10⁻⁶M, < 1×10⁻⁷M, < 1×10⁻⁸M, < 1×10⁻⁹M, or < 1×10⁻¹⁰M. The KD may be measured by BLI (Biolayer Interferometry, Octet) using immobilized bispecific protein against recombinant human CD40, his-tagged (Running Buffer: PBS, pH 7.4, 0.5% (v/v) Tween-20, Temp: 25°C).

In a preferred embodiment, the bispecific binding protein binds to MUC-1 with a dissociation constant (KD) of < 1×10⁻⁶M and to CD40 with a dissociation constant (KD) of < 1×10⁻⁶M. In a further preferred embodiment, the bispecific binding protein binds to MUC-1 with a dissociation constant (KD) of < 1×10⁻⁶M and to CD40 with a dissociation constant (KD) of < 1×10⁻⁷M.

In some embodiments, the bispecific binding protein of the present invention induces CD40 activation in dendritic cells or B cells in the presence of MUC-1 expressing tumor cells (e.g. derived from a tumor cell line expressing MUC-1) that is increased over the CD40 activation caused by an anti-CD40 antibody such as selicrelumab or sotigalimab. In some embodiments, the CD40 activation is increased by at least 50%, at least 60% or at least 70% compared to the CD40 activation caused by an anti-CD40 antibody (see assays described above).

### Anti-MUC-1 antibody

The present invention also provides an anti-MUC-1 antibody or an anti-MUC-1 antibody fragment. In some embodiment, the anti-MUC-1 antibody or anti-MUC-1 antibody fragment specifically binds to MUC-1. In some embodiments, the anti-MUC-1 antibody or the anti-MUC-1 antibody fragment comprises a VH CDR1 amino acid sequence according SEQ ID NO: 1, a VH CDR2 amino acid sequence according to SEQ ID NO: 2 and a VH CDR3 amino acid sequence according to SEQ ID NO: 3; and a VL CDR1 amino acid sequence according SEQ ID NO: 6, a VL CDR2 amino acid sequence according to SEQ ID NO: 7 and a VL CDR3 amino acid sequence according to SEQ ID NO: 8. In a further embodiment, the anti-MUC-1 antibody or the anti-MUC-1 antibody fragment comprises a variable heavy chain amino acid sequence having at least 60%, at least 70%, at least 80%, at least 90%, at least 95% or at least 99% sequence identity with SEQ ID NO: 4 and a variable light chain amino acid sequence having at least 60%, at least 70%, at least 80%, at least 90%, at least 95% or at least 99% sequence identity with SEQ ID NO: 9. In a further embodiment, the anti-MUC-1 antibody comprises a heavy chain amino acid sequence having at least 60%, at least 70%, at least 80%, at least 90%, at least 95% or at least 99% sequence identity with SEQ ID NO: 5 and a light chain amino acid sequence having at least 60%, at least 70%, at least 80%, at least 90%, at least 95% or at least 99% sequence identity with SEQ ID NO: 10. In some embodiments, the anti-MUC-1 antibody comprises a heavy chain amino acid sequence having at least 95% sequence identity with SEQ ID NO: 5 and a light chain amino acid sequence having at least 95% sequence identity with SEQ ID NO: 10. In some embodiments, the anti-MUC-1 antibody comprises a heavy chain amino acid sequence having at least 99% sequence identity with SEQ ID NO: 5 and a light chain amino acid sequence having at least 99% sequence identity with SEQ ID NO: 10. In some embodiments, the anti-MUC-1 antibody or the anti-MUC-1 antibody fragment binds to MUC-1 with a dissociation constant (KD) of < 1×10⁻⁶M, < 1×10⁻⁷M, < 1×10⁻⁸M, < 1×10⁻⁹M, or < 1×10⁻¹⁰M. In some embodiments, the anti-MUC-1 antibody or the anti-MUC-1 antibody fragment binds to MUC-1 with a dissociation constant (KD) of < 1×10⁻⁶M.

In one embodiment, the anti-MUC-1 antibody or anti-MUC-1 antibody fragment is an anti-MUC-1 antibody or an anti-MUC-1 antibody fragment as described herein in the context of the bispecific binding protein.

The embodiments described in the sections below relating to pharmaceutical compositions, medical uses and medical methods are also intended to be disclosed in the context of an anti-MUC-1 antibody or an anti-MUC-1 antibody fragment.

### CD40 VHH

The present invention also provides a VHH antibody, a nanobody or a single chain antibody binding to CD40. In some embodiments, the VHH, the nanobody or the single chain antibody specifically binds CD40. In a preferred embodiment, the present invention provides a VHH antibody binding CD40. In some embodiments, the anti-CD40 VHH comprises a CDR1 amino acid sequence according to SEQ ID NO: 12, a CDR2 amino acid sequence according to SEQ ID NO: 13 and a CDR3 amino acid sequence according to SEQ ID NO: 14. In a further embodiment, the anti-CD40 VHH comprises an amino acid sequence having at least 60%, at least 70%, at least 80%, at least 90%, at least 95% or at least 99% sequence identity with SEQ ID NO: 15. In some embodiments, the anti-CD40 VHH comprises an amino acid sequence having at least 90% sequence identity with SEQ ID NO: 15. In some embodiments, the anti-CD40 VHH comprises an amino acid sequence having at least 95% sequence identity with SEQ ID NO: 15. In some embodiments, the anti-CD40 VHH comprises an amino acid sequence having at least 99% sequence identity with SEQ ID NO: 15. In some embodiments, the CD40 VHH binds to CD40 with a dissociation constant (KD) of < 1×10⁻⁶M, < 1×10⁻⁷M, < 1×10⁻⁸M, < 1×10⁻⁹M, or < 1×10⁻¹⁰M. In some embodiments, the CD40 VHH binds to CD40 with a dissociation constant (KD) of < 1×10⁻⁶M. In some embodiments, the CD40 VHH binds to CD40 with a dissociation constant (KD) of < 1×10⁻⁷M.

In one embodiment, the anti-CD40 VHH antibody is an anti-CD40 VHH antibody as described herein in the context of the bispecific binding protein.

The embodiments described in the sections below relating to pharmaceutical compositions, medical uses and medical methods are also intended to be disclosed in the context of a VHH antibody binding CD40.

### Pharmaceutical compositions

In some embodiments, the bispecific binding protein is incorporated together with one or more pharmaceutically acceptable carriers into a pharmaceutical composition suitable for administration to a subject. Thus, the present disclosure relates to a pharmaceutical composition comprising the bispecific binding protein as described in any of the embodiments above, and one or more pharmaceutically acceptable carriers. Pharmaceutically acceptable carriers include any of a variety of solvents, dispersion media, coatings, antibacterial and antifungal agents, isotonic and absorption delaying agents, and the like that are physiologically compatible. Examples of pharmaceutically acceptable carriers include, but are not limited to, water, saline, phosphate buffered saline, dextrose, glycerol, ethanol and the like, as well as combinations thereof.

In some embodiments, the pharmaceutical composition comprises one or more tonicity agents or stabilizers. Non-limiting examples of such tonicity agents or stabilizers include sugars (e.g., sucrose), polyalcohols (e.g., mannitol or sorbitol), and sodium chloride.

In some embodiments, the pharmaceutical composition comprises one or more bulking agents and/or lyoprotectants (e.g., mannitol or glycine), buffers (e.g., phosphate, acetate, or histidine buffers), surfactants (e. g., polysorbates), antioxidants (e. g., methionine), and/or metal ions or chelating agents (e. g., ethylenediaminetetraacetic acid (EDTA)).

In some embodiments, the pharmaceutical composition comprises one or more auxiliary substances such as wetting or emulsifying agents, preservatives (e.g., benzyl alcohol) or buffers, which may enhance the shelf life and/or efficacy of the bispecific binding proteins disclosed herein.

Pharmaceutical compositions may be provided in any of a variety of forms. These include, for example, liquid, semi-solid and solid dosage forms, such as liquid solutions (e.g., injectable and infusible solutions), dispersions or suspensions, tablets, pills, powders, liposomes and suppositories. Suitability of certain forms may depend on the intended mode of administration and therapeutic application.

In some embodiments, the pharmaceutical composition is in the form of an injectable or infusible solution.

Pharmaceutical compositions are typically sterile and stable under conditions of manufacture, transport, and storage. Pharmaceutical compositions may be formulated as, for example, a solution, microemulsion, dispersion, liposome, or other ordered structure.

In some embodiments, a pharmaceutical composition is formulated as a structure particularly suitable for high drug concentration. For example, sterile injectable solutions can be prepared by incorporating a therapeutic agent in a desired amount in an appropriate solvent with one or a combination of ingredients enumerated herein, optionally followed by sterilization (e.g., filter sterilization). Generally, dispersions may be prepared by incorporating a bispecific binding protein into a sterile vehicle that contains a basic dispersion medium and other ingredient(s) such as those additional ingredients mentioned herein. In the case of sterile powders for the preparation of sterile injectable solutions, examples of preparation methods include vacuum drying and freeze-drying to yield a powder of the bispecific binding protein and any additional desired ingredient(s), e.g., from a previously sterile-filtered solution thereof.

Proper fluidity of a solution can be maintained, for example, by the use of a coating such as lecithin, by maintaining certain particle sizes (e.g., in the case of dispersions), and/or by using surfactants. Prolonged absorption of injectable compositions can be brought about, e.g., by including in the composition an agent that delays absorption (e.g., monostearate salts and/or gelatin).

### Medical uses and medical methods

In some embodiments, the bispecific binding protein or the pharmaceutical composition provided herein is for use in a method of treating a subject. In some embodiments, the bispecific binding protein or the pharmaceutical composition provided herein is for use in a method of treating cancer in a subject. In some embodiments, the subject is a human patient. In some embodiments, the subject is a mammal e.g. selected from a dog, a cat, a horse, a mouse and a rat.

In some embodiments, the present invention provides a method for treating a subject, wherein the method comprises administering the bispecific binding protein or the pharmaceutical composition in a therapeutically effective amount to a subject in need thereof. In some embodiments, the present invention provides a method for treating cancer in a subject, wherein the method comprises administering the bispecific binding protein or the pharmaceutical composition in a therapeutically effective amount to a subject in need thereof. In some embodiments, the subject is a human patient. In some embodiments, the subject is a mammal e.g. selected from a dog, a cat, a horse, a mouse and a rat.

In some embodiments, the present invention provides the use of a bispecific binding protein in the manufacture of a medicament. In some embodiments, the present invention provides the use of a bispecific binding protein in the manufacture of a medicament for treating cancer in a subject. In some embodiments, the subject is a human patient. In some embodiments, the subject is a mammal e.g. selected from a dog, a cat, a horse, a mouse and a rat.

In some embodiments, the cancer comprises a solid tumor. For example, the cancer may be selected from the group consisting of lung cancer, pancreatic cancer and colorectal cancer. In some embodiments, the cancer is lung cancer, preferably a non-small cell lung cancer (NSCLC). In some embodiments, the NSCLS is an NSCLC comprising an adenocarcinoma and/or a squamous cell carcinoma. Preferably, the NSCLC is an NSCLC of the adenocarcinoma type. In some embodiments, the cancer is pancreatic cancer, preferably pancreatic ductal adenocarcinoma (PDAC). In some embodiments, the cancer is colorectal cancer. In some embodiments, the solid tumor is metastatic.

In some embodiments, the cancer is selected from the group consisting of pancreatic ductal adenocarcinoma, non-small cell lung cancer (NSCLC) and colorectal cancer.

In some embodiments, the cancer involves overexpression of MUC-1 in the cancer cells. In some embodiments, the cancer is a cancer in which MUC-1 is overexpressed. In some embodiments, the tumor overexpresses MUC-1. Cancers that involve overexpression of MUC-1/cancers in which MUC-1 is overexpressed/tumors that overexpress MUC-1 are known to a skilled person. In some embodiments, a cancer that involves overexpression of MUC-1/a cancer in which MUC-1 is overexpressed/a tumor that overexpresses MUC-1 is identified in an assay according to Example 7 (i.e., preferably, cells are washed, blocked with human Immunoglobulin (e.g. Beroglobin), and stained with PE (phycoerythrin)-labeled anti-MUC-1 antibody (heavy chain SEQ ID NO: 5; light chain SEQ ID NO: 10); staining is quantified by flow cytometry; PE Quantum Beads are used to quantify MESF (Molecules of Equivalent Soluble Fluorochrome) values after anti-MUC-1 PE antibody binding to the cells; cells that show higher values for anti-MUC-1 binding (MESF) than HCC827 cells are considered as the cells of a cancer that involves overexpression of MUC-1/a cancer in which MUC-1 is overexpressed/a tumor that overexpresses MUC-1).

In some embodiments, administration of the bispecific binding protein or the pharmaceutical composition results in a measurable improvement in the subject. For example, this improvement may include any or any combination of tumor growth inhibition (TGI), tumor growth reduction, tumor regression, inhibition or reduction of metastases, improved survival, or improvement in any clinical sign indicative of cancer status or progression. Tumor growth may be assessed by measures such as, e.g., estimated or measured tumor volumes. In some embodiments, tumor growth inhibition or reduction is by at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% (e.g., based on lower tumor volume relative to a reference, such as a reference value representative of a tumor volume in a subject receiving no treatment). In some embodiments, administration results in regression of the tumor, i.e. a decrease in size of a tumor. This tumor regression may be partial (i.e., some of the tumor or cancer remains) or complete (e. g., the tumor volume reaches approximately zero and/or the tumor is no longer measurable or detectable).

In some embodiments, the bispecific binding protein or the pharmaceutical composition provided herein is for use in treating a T cell dysfunctional disease. In some embodiments, the T cell dysfunctional disease is unresolved acute infection, chronic infection and tumor immunity.

The medical uses and medical method provided herein may include first line, second line, third line or further lines of treatment of patients. The cancer may be metastatic, recurrent, relapsed, resistant or refractory to one or more anti-cancer treatments. Thus, the patients may be treatment naive, or may have received one or more previous anti-cancer therapies, which have not completely cured the disease.

### Methods, nucleic acid, host cells

The present invention also provides a nucleic acid encoding a bispecific binding protein as described herein. In some embodiments, the nucleic acid involves several separate nucleic acids. For example, the heavy and light chain of the bispecific binding protein may be encoded by separate nucleic acids. In some embodiments, the present invention provides a vector or a set of vectors comprising the nucleic acid. The vector or set of vectors may include suitable promotor sequences and/or regulatory elements.

Also provided is host cell comprising the nucleic acid or the vector described herein. The host cell may be any host cell suitable for protein expression including, but not limited to CHO cells, 239T cells, COS cells, SP2O cells and Sf9 cells.

The present invention also provides a method for preparing a bispecific binding protein as described herein. In some embodiments, the method involves the steps of culturing a host cell as described herein under conditions suitable for protein expression and purifying the bispecific protein. Purification may include any protein purification methods established in the art.

The present invention is *inter alia* described by the following embodiments:
Embodiment 1: A bispecific binding protein binding MUC-1 and CD40.
Embodiment 2: The bispecific binding protein of embodiment 1, wherein the bispecific binding protein comprises at least one binding site for MUC-1 and at least one binding site for CD40.
Embodiment 3: The bispecific binding protein of embodiment 1 or embodiment 2, wherein the bispecific binding protein comprises two binding sites for MUC-1 and two binding sites for CD40.
Embodiment 4: The bispecific binding protein of any one of embodiments 1-3, wherein the two binding sites for MUC-1 are identical and/or wherein the two binding sites for CD40 are identical.
Embodiment 5: The bispecific binding protein of any one of embodiments 1-4, wherein the bispecific binding protein comprises an anti-MUC-1 antibody or an anti-MUC-1 antibody fragment.
Embodiment 6: The bispecific binding protein of any one of embodiments 1-5, wherein the bispecific binding protein comprises one, two or more anti-CD40 VHHs.
Embodiment 7: The bispecific binding protein of any one of embodiments 1-6, wherein the bispecific binding protein comprises one anti-MUC-1 antibody and two anti-CD40 VHHs.
Embodiment 8: The bispecific binding protein of any one of embodiments 5-7, wherein the anti-MUC-1 antibody or the anti-MUC-1 antibody fragment comprises a VH CDR1 amino acid sequence according SEQ ID NO: 1, a VH CDR2 amino acid sequence according to SEQ ID NO: 2 and a VH CDR3 amino acid sequence according to SEQ ID NO: 3; and a VL CDR1 amino acid sequence according SEQ ID NO: 6, a VL CDR2 amino acid sequence according to SEQ ID NO: 7 and a VL CDR3 amino acid sequence according to SEQ ID NO: 8.
Embodiment 9: The bispecific binding protein of any one of embodiments 6-8, wherein the anti-CD40 VHH comprises a CDR1 amino acid sequence according to SEQ ID NO: 12, a CDR2 amino acid sequence according to SEQ ID NO: 13 and a CDR3 amino acid sequence according to SEQ ID NO: 14.
Embodiment 10: The bispecific binding protein of any one of embodiments 5-9, wherein the anti-MUC-1 antibody or the MUC-1 antibody fragment comprises a variable heavy chain amino acid sequence having at least 80%, at least 90%, at least 95% or at least 99% sequence identity with SEQ ID NO: 4 and/or a variable light chain amino acid sequence having at least 80%, at least 90%, at least 95% or at least 99% sequence identity with SEQ ID NO: 9.
Embodiment 11: The bispecific binding protein of any one of embodiments 5-9, wherein the anti-MUC-1 antibody or the MUC-1 antibody fragment comprises
   (a) a variable heavy chain amino acid sequence having at least 60% sequence identity with SEQ ID NO: 4 and a variable light chain amino acid sequence having at least 60% sequence identity with SEQ ID NO: 9,
   (b) a variable heavy chain amino acid sequence having at least 70% sequence identity with SEQ ID NO: 4 and a variable light chain amino acid sequence having at least 70% sequence identity with SEQ ID NO: 9,
   (c) a variable heavy chain amino acid sequence having at least 80% sequence identity with SEQ ID NO: 4 and a variable light chain amino acid sequence having at least 80% sequence identity with SEQ ID NO: 9,
   (d) a variable heavy chain amino acid sequence having at least 90% sequence identity with SEQ ID NO: 4 and a variable light chain amino acid sequence having at least 90% sequence identity with SEQ ID NO: 9,
   (e) a variable heavy chain amino acid sequence having at least 95% sequence identity with SEQ ID NO: 4 and a variable light chain amino acid sequence having at least 95% sequence identity with SEQ ID NO: 9, or
   (f) a variable heavy chain amino acid sequence having at least 99% sequence identity with SEQ ID NO: 4 and a variable light chain amino acid sequence having at least 99% sequence identity with SEQ ID NO: 9.
Embodiment 12: The bispecific binding protein of any one of embodiments 5-10, wherein the bispecific binding protein exhibits at least 80%, at least 90% or at least 95% of the binding activity of an anti-MUC-1 antibody with a variable heavy chain amino acid sequence of SEQ ID NO: 4 and a variable light chain amino acid sequence of SEQ ID NO: 9.
Embodiment 13: The bispecific binding protein of any one of embodiments 5-12, wherein the anti MUC-1 antibody comprises a heavy chain amino acid sequence having at least 80%, at least 90%, at least 95% or at least 99% sequence identity with SEQ ID NO: 5 and/or a light chain amino acid sequence having at least 80%, at least 90%, at least 95% or at least 99% sequence identity with SEQ ID NO: 10.
Embodiment 14: The bispecific binding protein of any one of embodiments 5-13, wherein the anti-MUC-1 antibody or the MUC-1 antibody fragment comprises
   (a) a heavy chain amino acid sequence having at least 60% sequence identity with SEQ ID NO: 5 and a light chain amino acid sequence having at least 60% sequence identity with SEQ ID NO: 10,
   (b) a heavy chain amino acid sequence having at least 70% sequence identity with SEQ ID NO: 5 and a light chain amino acid sequence having at least 70% sequence identity with SEQ ID NO: 10,
   (c) a heavy chain amino acid sequence having at least 80% sequence identity with SEQ ID NO: 5 and a light chain amino acid sequence having at least 80% sequence identity with SEQ ID NO: 10,
   (d) a heavy chain amino acid sequence having at least 90% sequence identity with SEQ ID NO: 5 and a light chain amino acid sequence having at least 90% sequence identity with SEQ ID NO: 10,
   (e) a heavy chain amino acid sequence having at least 95% sequence identity with SEQ ID NO: 5 and a light chain amino acid sequence having at least 95% sequence identity with SEQ ID NO: 10, or
   (f) a heavy chain amino acid sequence having at least 99% sequence identity with SEQ ID NO: 5 and a light chain amino acid sequence having at least 99% sequence identity with SEQ ID NO: 10.
Embodiment 15: The bispecific binding protein of any one of embodiments 5-14, wherein the bispecific binding protein exhibits at least 80%, at least 90% or at least 95% of the binding activity of an anti-MUC-1 antibody with a heavy chain amino acid sequence of SEQ ID NO: 5 and a light chain amino acid sequence of SEQ ID NO: 10.
Embodiment 16: The bispecific binding protein of any one of embodiments 6-15, wherein the anti-CD40 VHH comprises an amino acid sequence having at least 80%, at least 90%, at least 95% or at least 99% sequence identity with SEQ ID NO: 15.
Embodiment 17: The bispecific binding protein of any one of embodiments 6-16, wherein the bispecific binding protein exhibits at least 80%, at least 90% or at least 95% of the binding activity of an anti-CD40 VHH with an amino acid sequence of SEQ ID NO: 15.
Embodiment 18: The bispecific binding protein of any one of embodiments 6-18, wherein the anti-MUC-1 antibody or the anti-MUC-1 antibody fragment and the anti-CD40 VHH are directly liked in a fusion protein with or without an amino acid linker sequence.
Embodiment 19: The bispecific binding protein of embodiment 18, wherein the anti-MUC-1 antibody or the MUC-1 antibody fragment and the anti-CD40 VHH are directly liked in a fusion protein with an amino acid linker sequence.
Embodiment 20: The bispecific binding protein of embodiment 19, wherein the amino acid linker sequence comprises 10-30 amino acids and/or wherein the amino acids are selected from G and S.
Embodiment 21: The bispecific binding protein of embodiment 18 or embodiment 19, wherein the amino acid linker sequence comprises an amino acid sequence according to SEQ ID NO: 11.
Embodiment 22: The bispecific binding protein of any one of embodiments 18-21, wherein the anti-CD40 VHH is linked to the C-terminus of the anti-MUC-1 antibody or the MUC-1 antibody fragment.
Embodiment 23: A bispecific binding protein comprising a light chain amino acid sequence according to SEQ ID NO: 10 and a heavy chain amino acid sequence according to SEQ ID NO: 16.
Embodiment 24: A bispecific binding protein binding to MUC-1 and CD40, wherein the bispecific binding protein comprises a light chain amino acid sequence according to SEQ ID NO: 10 and a heavy chain amino acid sequence according to SEQ ID NO: 16.
Embodiment 25: The bispecific binding protein of embodiment 23 or embodiment 24, wherein the bispecific binding protein comprises two light chain amino acid sequences according to SEQ ID NO: 10 and two heavy chain amino acid sequences according to SEQ ID NO: 16.
Embodiment 26: The bispecific binding protein of embodiment 25, wherein the two heavy chain amino acid sequences are linked via a disulphide bridge.
Embodiment 27: The bispecific binding protein of embodiment 26, wherein the two heavy chain amino acid sequences are linked via two disulphide bridges.
Embodiment 28: The bispecific binding protein of any one of embodiments 1-27, wherein the bispecific binding protein binds to MUC 1 with a dissociation constant (KD) of < 1×10⁻⁶M.
Embodiment 29: The bispecific binding protein of any one of embodiments 1-28, wherein the bispecific binding protein binds to CD40 with a dissociation constant (KD) of < 1×10⁻⁶M.
Embodiment 30: The bispecific binding protein of any one of embodiments 1-29, wherein the bispecific binding protein binds to CD40 with a dissociation constant (KD) of < 1×10⁻⁷M.
Embodiment 31: The bispecific binding protein of any one of embodiments 1-30, wherein the bispecific binding protein induces CD40 activation on B cells in the presence of MUC-1-expressing tumor cells.
Embodiment 32: The bispecific binding protein of any one of embodiments 1-30, wherein the bispecific binding protein does not induce CD40 activation on B cells in the absence of MUC-1-expressing tumor cells or in the absence of MUC-1 antigen.
Embodiment 33: The bispecific binding protein of any one of embodiments 1-30, wherein the bispecific binding protein induces CD40 activation on dendritic cells in the presence of MUC-1-expressing tumor cells.
Embodiment 34: The bispecific binding protein of any one of embodiments 1-30, wherein the bispecific binding protein does not induce CD40 activation on dendritic cells in the absence of MUC-1-expressing tumor cells or in the absence of MUC-1 antigen.
Embodiment 35: The bispecific binding protein of any one of embodiments 1-30, wherein the bispecific binding protein does not induce CD40 activation on dendritic cells in the presence MUC-1 negative cells.
Embodiment 36: The bispecific binding protein of any one of embodiments 1-35 for use in therapy.
Embodiment 37: The bispecific binding protein of any one of embodiments 1-35 for use in the treatment of cancer.
Embodiment 38: The bispecific binding protein of any one of embodiments 1-35 for use in the treatment of a solid tumor.
Embodiment 39: The bispecific binding protein of any one of embodiments 1-35 for use in the treatment of lung cancer (preferably NSCLC), pancreatic cancer (preferably PDAC) and colorectal cancer.
Embodiment 40: A nucleic acid encoding the bispecific binding protein of any one of embodiments 1-35.
Embodiment 41: A vector or a set of vectors comprising the nucleic acid of embodiment 40.
Embodiment 42. A host cell comprising the nucleic acid of embodiment 40 or the vector or set of vectors of embodiment 41.
Embodiment 43: A method for preparing a bispecific binding protein of any one of embodiments 1-35.

### Examples

The working examples presented below are intended to illustrate particular embodiments of the invention and are not intended to limit the scope of the specification or the claims in any way.

### Example 1: Antibody production

The antibody fusion protein FP1 as shown in Figure 14 was produced. Specifically, nucleotides for the recombinant expression of the heavy chain (SEQ ID NO: 16) and the light chain (SEQ ID NO: 10) of the fusion protein were cloned into mammalian expression vectors (pTT5 (Canadian Research Council)), allowing for the transient expression in Expi293 or ExpiCHO cells according to the manufacturer's instructions (Thermo Fisher Scientific). Antibody-containing supernatants were harvested by centrifugation five days post transfection prior to purification via MabSelect antibody purification chromatography resin (GE Healthcare). Afterwards, buffer exchange to phosphate-buffered saline (PBS), pH 6.8 was performed overnight using Pur-A-LyzerTM Maxi 3500 Dialysis Kit (Sigma Aldrich). After sterile filtration with Ultrafree^{®}-CL GV 0.22 µm centrifugal devices (Merck Millipore), the antibody concentrations were measured using Nanodrop ND-1000 (Peqlab). Analytical SEC analyses were conducted to determine sample purities (% target monomer peaks) using 7.5 µg protein per sample on a TSKgel UP-SW3000 column (2 µm, 4.6 × 300 mm, Tosoh Bioscience) in an Agilent HPLC 1260 Infinity system with a flow rate of 0.35 ml/min. Visualization of protein purities and verification of molecular masses were performed via SDS-PAGE (Thermo Fisher Scientific) under reducing and non-reducing conditions, loading 1 µg prepared protein sample per lane (4-12% Bis-Tris gel), respectively, and separating for 90 min at 200 V prior Coomassie staining.

### DNA sequence

### Heavy chain:

- Nucleotide sequence of anti-MUC1-anti-CD40-VHH heavy chain was adapted and cloned into an expression vector
- Nucleotide sequence corresponding to leader sequence is underlined
- Start and stop codons are in bold

SEQ ID NO:17:
ATGGAAACAGACACCCTGCTGCTGTGGGTGCTGCTGCTGTGGGTGCCCGGCTCCACAGGCGAGGTGCA
GCTGGTGCAGTCTGGGGCCGAGGTGAAGAAACCAGGCGCCAGCGTGAAGGTGTCCTGCAAGGCCAGCG
GCTACAGCTTCACCGGCCACTACATGCACTGGGTGCGCCAGGCTCCAGGCCAGGGACTGGAATGGATGG
GCTGGATCGACCCTGTGACCGGCGGCACCAAATACGCCCAGAAGTTCCAGGGCTGGGTCACCATGACCC
GGGACACCAGCATCAGCACCGCCTACATGGAACTGAGCCGGCTGAGAAGCGACGACACCGCCGTGTACT
ACTGCGCCAGAGAAGTGACAGGCGACCGGGGCCAGTTCGATAAGTGGGGCCAGGGCACACTCGTGACC
GTGAGCAGCGCCTCCACCAAGGGCCCATCGGTCTTCCCCCTGGCACCCTCCTCCAAGAGCACCTCTGGG
GGCACAGCGGCCCTGGGCTGCCTGGTCAAGGACTACTTCCCCGAACCGGTGACGGTGTCGTGGAACTCA
GGCGCCCTGACCAGCGGCGTGCACACCTTCCCGGCTGTCCTACAGTCCTCAGGACTCTACTCCCTCAGC
AGCGTGGTGACCGTGCCCTCCAGCAGCTTGGGCACCCAGACCTACATCTGCAACGTGAATCACAAGCCC
AGCAACACCAAGGTGGACAAGAGAGTTGAACCCAAGAGCTGCGACAAGACCCACACCTGTCCTCCATGTC
CTGCTCCTGAAGCTGCCGGGGGCCCTTCCGTGTTTCTGTTCCCTCCAAAGCCTAAGGACACCCTGATGAT
CAGCAGAACCCCTGAAGTGACCTGCGTGGTGGTGGATGTGTCTCACGAGGACCCCGAAGTGAAGTTCAA
TTGGTACGTGGACGGCGTGGAAGTTCACAACGCAAAGACTAAACCTCGAGAAGAGCAGTATAATTCTACA
TATCGTGTCGTGTCCGTTCTCACCGTGCTCCACCAGGATTGGTTGAATGGCAAAGAGTATAAGTGCAAGG

TGTCCAACAAAGCACTTCCAGCACCCATCGAAAAGACCATTTCTAAGGCCAAGGGGCAACCCCGAGAACC
TCAAGTGTATACTCTGCCCCCTTCTCGCGAAGAGATGACTAAAAACCAGGTCAGTCTTACTTGCTTGGTCA
AAGGCTTCTACCCTAGCGACATCGCCGTCGAATGGGAAAGCAATGGGCAACCAGAAAATAACTACAAGAC
AACCCCACCTGTCCTCGATTCAGACGGCAGTTTTTTTCTGTATAGTAAACTGACAGTGGACAAAAGCCGCT
GGCAGCAGGGTAACGTCTTTTCATGTTCAGTTATGCATGAGGCACTTCACAATCATTACACCCAAAAGAGC
CTGTCTCTCAGTCCAGGTGGTAGTGGTGGCGGGTCTGGAGGCTCAGGCGGGGGTGGTTCAGGGGAAGT
ACAGCTCGTAGAATCAGGAGGAGGGGTGGTCCAACCAGGCGGCTCACTGCGACTGTCATGCGCAGCTTC
AGGATTCACATTCAGCTCTGCAACCATGGGCTGGTTTAGGCAAGCCCCCGGAAAGGGGCTCGAATGGGT
CGCATACATTTCATCCGTAGGGGGGGGTACTGATTACGCAGACTCAGTAAAGGGACGTTTTACAATATCTC
GCGACAATGCAAAAAATACACTGTATCTTCAAATGAACTCACTCAGGGCTGAGGATACCGCCGTATATTAC
TGTGTCACAATGCGGTCAATACGTAACCCAGACTTTGGGTCACGGGGGCAAGGGACACTCGTGACAGTTT
CATCTGCATAATGA

### Light chain:

- Nucleotide sequence of anti-MUC1 lambda light chain was adapted and cloned into an expression vector.
- Nucleotide sequence corresponding to leader sequence is underlined
- Start and stop codons are in bold

SEQ ID NO: 18:
ATGCGGGCTCTCCTGGCCAGGCTGCTGCTGTGCGTGCTGGTGGTGAGCGACAGCAAGGGCAGCTACGT
GCTGACTCAGCCTCCTAGCGTCAGCGTGGCTCCCGGCAAAACTGCTAGGATCACATGTGGCGGCAACAA
CATCGGCAGCAAGAGCGTGCACTGGTATCAGCAGAAGCCCGGCCAAGCCCCAGTGCTCGTGATCTACTA
CGGCAGCAATAGGCCTTCCGGCATCCCAGAGAGATTCTCCGGCAGCAACTCCGGCAATACTGCCACACT
GACAATCTCTAGGGTGGAGGCCGGAGACGAGGCTGACTACTATTGCCAAGTGTGGGACAGCAGCTCCGA
CTGGGTGTTCGGCGGAGGCACTAAGCTGACTGTGCTGGGACAGCCTAAGGCCGCTCCTAGCGTGACTCT
GTTCCCACCAAGCAGCGAGGAGCTGCAAGCCAATAAAGCCACTCTGGTGTGTCTGATCAGCGATTTCTAC
CCCGGCGCCGTGACAGTCGCTTGGAAGGCTGATTCCAGCCCAGTGAAGGCCGGCGTGGAGACTACAACT
CCTTCCAAGCAGTCCAACAACAAGTACGCCGCCAGCTCCTATCTGTCTCTGACTCCAGAGCAGTGGAAGA
GCCACAAGAGCTACAGCTGCCAAGTGACTCACGAGGGCTCCACTGTGGAGAAGACAGTGGCCCCTACAG
AGTGCAGCTGATAG

### Protein sequence

### Heavy chain:

- Amino acid sequence of anti-MUC1-anti-CD40-VHH heavy chain
- Signal peptide is underlined

SEQ ID NO: 19:
METDTLLLWVLLLWVPGSTGEVQLVQSGAEVKKPGASVKVSCKASGYSFTGHYMHWVRQAPGQGLEWMGW
IDPVTGGTKYAQKFQGWVTMTRDTSISTAYMELSRLRSDDTAVYYCAREVTGDRGQFDKWGQGTLVTVSSAS
TKGPSVFPLAPSSKSTSGGTAALGCLVKDYFPEPVTVSWNSGALTSGVHTFPAVLQSSGLYSLSSVVTVPSSS
LGTQTYICNVNHKPSNTKVDKRVEPKSCDKTHTCPPCPAPEAAGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPVLDSDGSFFLYSKLTV

DKSRWQQGNVFSCSVMHEALHNHYTQKSLSLSPGGSGGGSGGSGGGGSGEVQLVESGGGVVQPGGSLRL
SCAASGFTFSSATMGWFRQAPGKGLEWVAYISSVGGGTDYADSVKGRFTISRDNAKNTLYLQMNSLRAEDTA
VYYCVTMRSIRNPDFGSRGQGTLVTVSSA

### Light chain:

- Amino acid sequence of anti-MUC1 light chain
- Signal peptide is underlined

SEQ ID NO:20:

MRALLARLLLCVLVVSDSKGSYVLTQPPSVSVAPGKTARITCGGNNIGSKSVHWYQQKPGQAPVLVIYYGSNR

PSGIPERFSGSNSGNTATLTISRVEAGDEADYYCQVWDSSSDWVFGGGTKLTVLGQPKAAPSVTLFPPSSEEL

QANKATLVCLISDFYPGAVTVAWKADSSPVKAGVETTTPSKQSNNKYAASSYLSLTPEQWKSHKSYSCQVTHE

GSTVEKTVAPTECS

Further characterization showed that, surprisingly, the CD40 VHH of FP1 does not compete with the natural CD40L ligand for binding to CD40 (as shown e.g. by epitope binning or Octet-Biolayer Interferometry). In line with this finding, FP1 does not cause competition with the natural CD40L signal.

Moreover, it was found that the CD40 VHH of FP1 (and FP1) is cross-reactive with cynomolgus monkey CD40.

Moreover, it was observed that the CD40 VHH of FP1 causes strictly conditional agonism (i.e. an antibody fusion protein that does not have MUC-1 binding activity, but in which two CD40 VHHs of FP1 are linked to the C terminus of an antibody do not cause agonism). In contrast, a corresponding antibody fusion protein with reference CD40 antibodies (like selicrelumab or sotigalimab) does not show strictly conditional agonism, even if used bivalently. Thus, the CD40 VHH of FP1 shows strong activation in MUC-1-expressing tumor cells, but no/almost no activation in a MUC-1 negative context, whereas reference CD40 antibodies (i.e. selicrelumab or sotigalimab) do not show conditional activity.

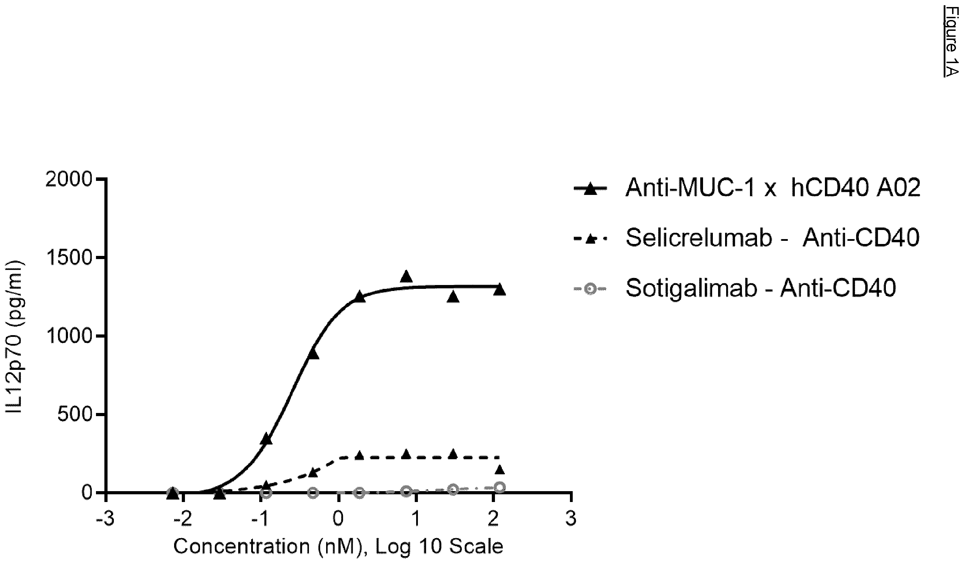

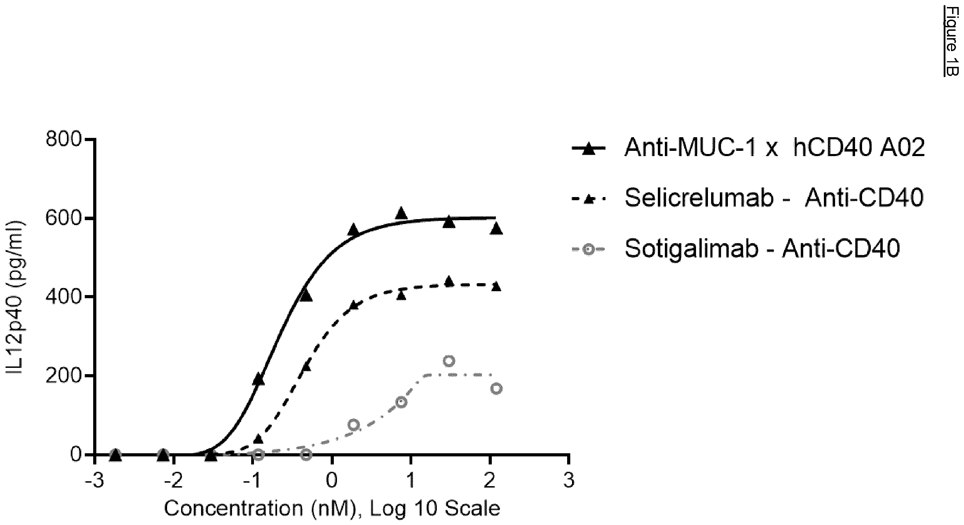

### Example 2: In vitro activity of MUC-1 conditional CD40 agonist (anti-MUC-1 × hCD40 A02, Fig. 1)

Human peripheral blood mononuclear cells (PBMCs) were purified out of whole blood from healthy donors using a ficoll gradient (Pancoll human / PAN Biotech). After washing of cells using Dulbecco's PBS (PAN Biotech), PBMCs were counted, and a cell suspension prepared for magnetic cell separation. The Human Monocyte Enrichment Kit without CD16 Depletion (Stemcell Technologies) was used to isolate untouched monocytes out of PBMCs via negative selection. Isolated monocytes were counted and adjusted for differentiation into monocyte-derived immature dendritic cells using 200 ng/ml GM-CSF and 50 ng/ml IL-4 (both Peprotech) in RPMI1640 medium (PAN Biotech) supplemented with 10% Fetal Bovine Serum (PAN Biotech), 2 mM L-Glutamine (PAN Biotech), 1x Penicillin/Streptomycin (PAN Biotech). HCC-827 tumor cells were counted (50 000 cells per 96 U-bottom well) and co-cultured with monocyte-derived immature dendritic cells (50 000 cells per 96 U-bottom well). Escalating doses of CD40 agonists (Anti-MUC-1 × hCD40, selicrelumab or sotigalimab) were added to the co-cultures and incubated for 24 hours. The supernatants of the co-cultures were collected for further analysis of IL-12 cytokines (p40 or p70) using commercially available IL-12 ELISA kits (Thermo Fischer). The graph displays IL-12p40 or IL-12p70 levels after treatment of dendritic cell HCC827 co-cultures with CD40 agonists (anti-MUC-1 × hCD40, selicrelumab or sotigalimab).

As can be seen from Fig. 1, in the presence of HCC-827 cells, which overexpress MUC-1, anti-MUC-1 × CD40 causes strong activation of CD40. A lower level of activation was observed with selicrelumab or sotigalimab.

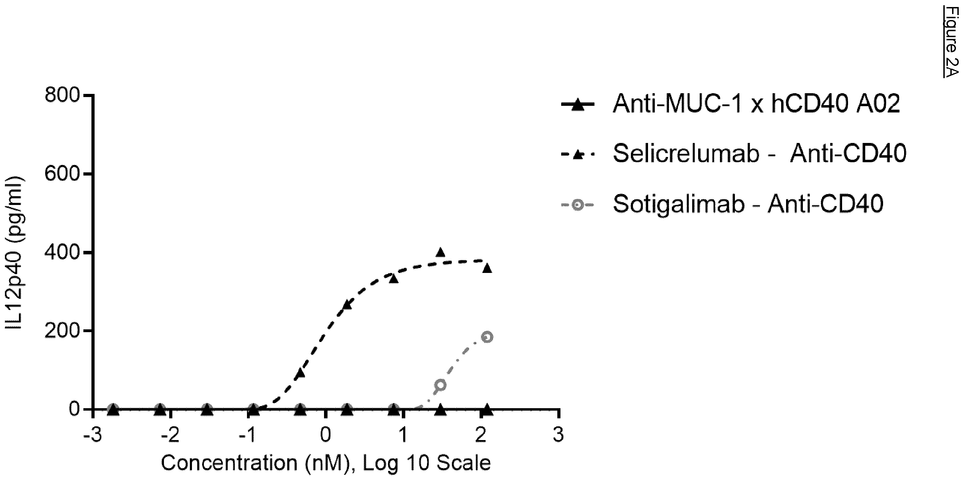

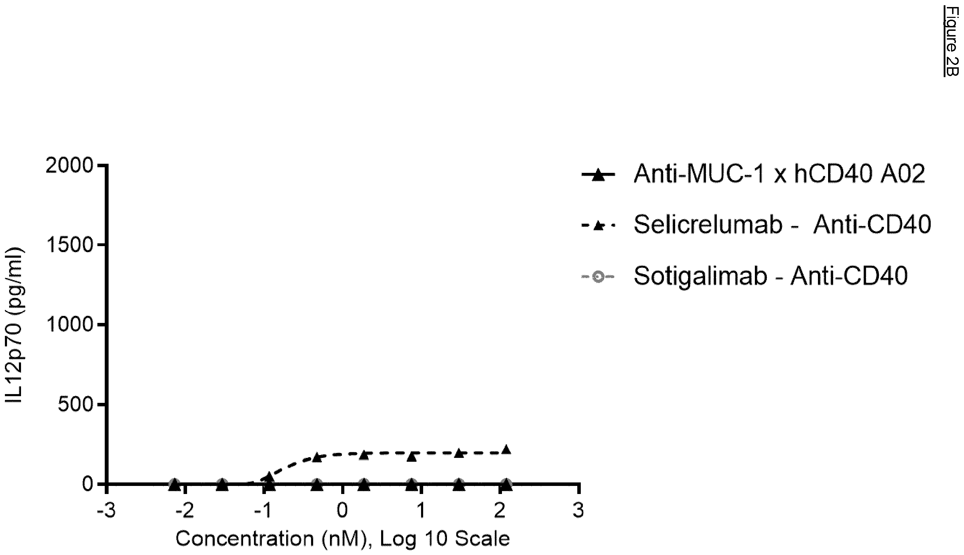

### Example 3: No activation of CD40 by anti-MUC-1 × hCD40 A02 in the absence of MUC-1 expressing tumor cells (Fig. 2)

Human peripheral blood mononuclear cells (PBMCs) were purified out of whole blood from healthy donors using a ficoll gradient (Pancoll human / PAN Biotech). After washing of cells using Dulbecco's PBS (PAN Biotech), PBMCs were counted and a cell suspension prepared for magnetic cell separation. The Human Monocyte Enrichment Kit without CD16 Depletion (STEMCELL Technologies) was used to isolate untouched monocytes out of PBMCs via negative selection. Isolated monocytes were counted and adjusted for differentiation into monocyte-derived immature dendritic cells using 200 ng/ml GM-CSF and 50 ng/ml IL-4 (both Peprotech) in RPMI1640 medium supplemented with 10% Fetal Bovine Serum (PAN Biotech), 2 mM L-Glutamine (PAN Biotech), 1x Penicillin/Streptomycin (PAN Biotech). Monocyte-derived immature dendritic cells (50 000 cells per 96 U-bottom well) were treated for 24 hours with escalating doses of CD40 agonists (anti-MUC-1 × hCD40 A02, selicrelumab or sotigalimab). The supernatant of the dendritic cell only culture was collected for further analysis of IL-12 cytokines (p40 or p70) using commercially available IL-12 ELISA kits (Thermo Fischer). The graph displays IL-12p40 or IL-12p70 levels after treatment of dendritic cell with CD40 agonists (Anti-MUC-1 × hCD40, selicrelumab or sotigalimab).

As can be seen from Fig. 2, no activation of CD40 occurred with anti-MUC-1 × CD40 in the absence of MUC-1 overexpressing HCC-827 cancer cells. In contrast, with selicrelumab or sotigalimab still some activation was observed even in the absence of MUC-1 overexpressing cancer cells.

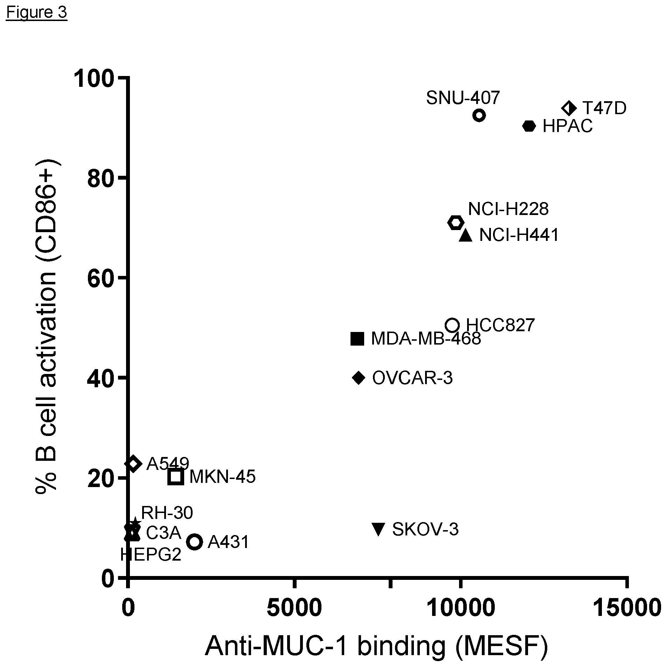

### Example 4: Magnitude of B cell activation by anti-MUC-1 × hCD40 A02 depends on anti-MUC-1 binding to tumor cells (Fig. 3)

Human peripheral blood mononuclear cells (PBMCs) were purified out of whole blood from healthy donors using a ficoll gradient (Pancoll human / PAN Biotech). After washing of cells using Dulbecco's PBS (PAN Biotech), PBMCs were counted, and a cell suspension prepared for magnetic cell separation. The Easysep B Cell Isolation Kit (Stemcell Technologies) was used to isolate fresh B cells out of PBMCs. Isolated B cells were counted and adjusted for the following co-culture experiment. RPMI1640 medium supplemented with 10% Fetal Bovine Serum (PAN Biotech), 2 mM L-Glutamine (PAN Biotech), 1x Penicillin/Streptomycin (PAN Biotech) was used as culture media. Tumor cells (50.000 cells per 96 U-bottom well) from various tumor cell lines (T47D, SNU047, HPAC, NCI-H441, NCI-H228, HCC827, MDA-MB-468, OVCAR3, SKOV3, MKN45, A549m A431, RH-30, C3A) and isolated B cells (50.000 cells per 96 U-bottom well) were co-cultured for 24 hours. Drug treatment was added at the beginning of the co-culture period. After 24 hours, suspension cells were harvested for flow cytometry analysis of CD80 and CD86 surface expression. For detection of anti-MUC-1 binding on tumor cells, various tumor cell lines (T47D, SNU047, HPAC, NCI-H441, NCI-H228, HCC827, MDA-MB-468, OVCAR3, SKOV3, MKN45, A549m A431, RH-30, C3A) were enzymatically detached with accutase (PAN Biotech), and 100 000 cells seeded into each 96 well of a V-bottom plate. Cells were washed, blocked with beriglobin (Behring CSL), and stained with anti-MUC-1 PE-labeled antibody. Stained cells were measured via flow cytometry in an LSR II Fortessa (BD Biosciences). PE Quantum Beads (Bangs Laboratories) were used to quantify MESF (Molecules of Equivalent Soluble Fluorochrome) values after anti-MUC-1 PE antibody binding to each cell line using bead manufacturers standard protocol. The graph displays the percent of B cell activation after co-culture with an indicated cell line and its correlation with anti-MUC-1 binding to the respective cell line.

As can be seen from Fig. 3, the magnitude of B cell activation depends on anti-MUC-1 binding to the tumor cells.

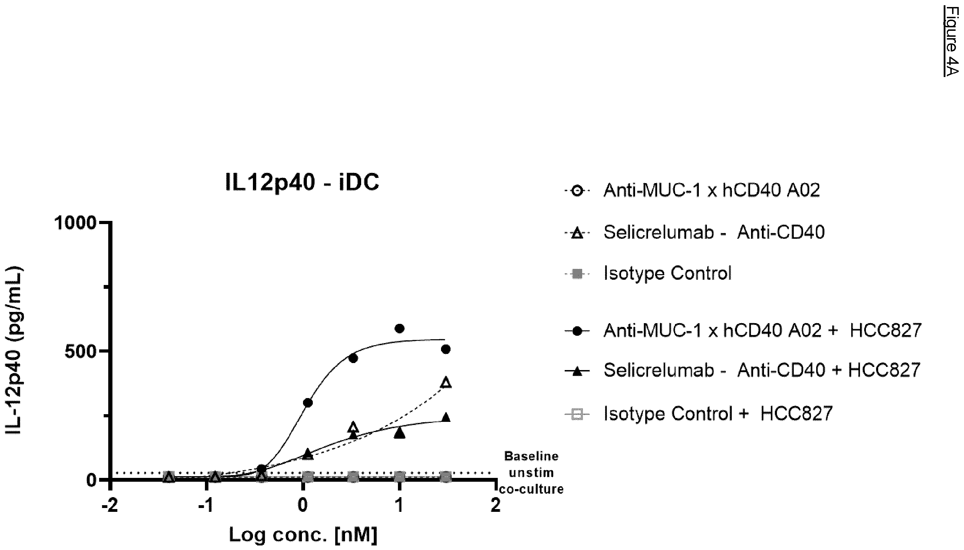

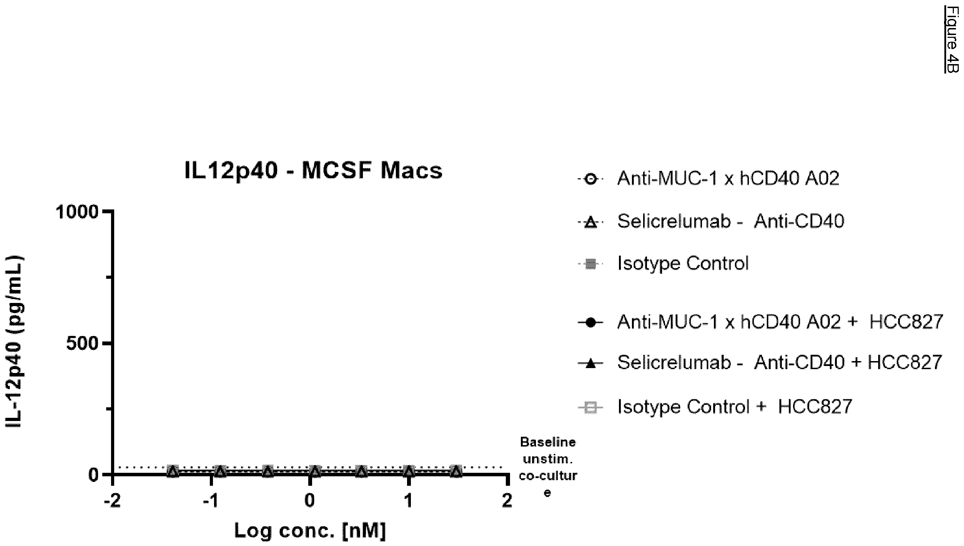

### Example 5: MUC-1 conditional agonist targeting human CD40 only induces IL-12p40 in iDCs but not M-CSF macrophages (Fig. 4)

Peripheral blood mononuclear cells (PBMCs) were purified out of whole blood from healthy donors using a ficoll gradient (Pancoll human / PAN Biotech). After washing of cells using Dulbecco's PBS (PAN Biotech), PBMCs were counted and a cell suspension prepared for magnetic cell separation. Human monocyte Enrichment Kit without CD16 Depletion (Stemcell Technologies) was used to isolate untouched monocytes out of PBMCs via negative selection. Isolated monocytes were counted and adjusted for differentiation into monocyte-derived M-CSF macrophages using 100 ng/ml M-CSF (Peprotech) in RPMI1640 medium supplemented with 10% Fetal Bovine Serum (PAN Biotech), 2 mM L-Glutamine (PAN Biotech), 1x Penicillin/Streptomycin (PAN Biotech). Alternatively, isolated monocytes were counted and adjusted for differentiation into monocyte-derived immature dendritic cells using 200 ng/ml GM-CSF and 50 ng/ml IL-4 (both Peprotech) in RPMI1640 medium supplemented with 10% Fetal Bovine Serum (PAN Biotech), 2 mM L-Glutamine (PAN Biotech), 1x Penicillin/Streptomycin (PAN Biotech). Monocyte-derived M-CSF macrophages or monocyte-derived immature dendritic cells (50.000 cells per 96 U-bottom well) were co-cultured with HCC-827 lung cancer cells for 24 hours. Treatments were added as indicated at the beginning of the co-culture period. The supernatants of the co-cultures were collected for further analysis of IL-12 cytokine content using a commercially available IL-12 ELISA kit (Thermo Fischer). The graph displays IL-12p40 induction in macrophage or dendritic cells after treatment with CD40 agonists (Anti-MUC-1 × hCD40 A02, selicrelumab) and Isotype Control antibody.

As can be seen from Fig. 4, the MUC-1 conditional agonist anti-MUC-1 × CD40 activates CD40 only in immature Dendritic Cells (iDCs), but not macrophage colony-stimulating factor (M-CSF) macrophages.

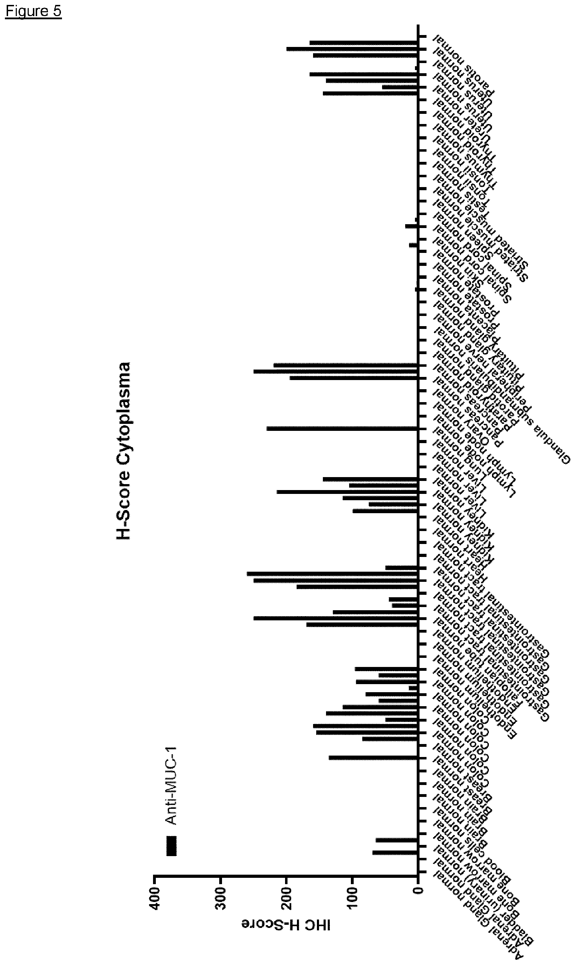

### Example 6: Kidney and Lung express high levels of MUC-1 (Fig. 5)

FDA Normal Tissue Panel TMAs (mandatory including lung, kidney and colon) were stained using anti-MUC-1 antibody clone (D11-28) in addition to the respective isotype control. Total MUC-1 protein expression was analyzed by IHC staining. Staining positivity was assessed for each compartment (membranous, cytoplasmic and nuclear) separately employing H-Scoring. The individual values (% membranous / cytoplasmic / nuclear positivity with 1+ / 2+ / 3+ intensity) contributing to the H-Score as well as the calculated H-scores for each compartment. Cytoplamic H-Scores of various tissues were quantified and displayed in the figure.

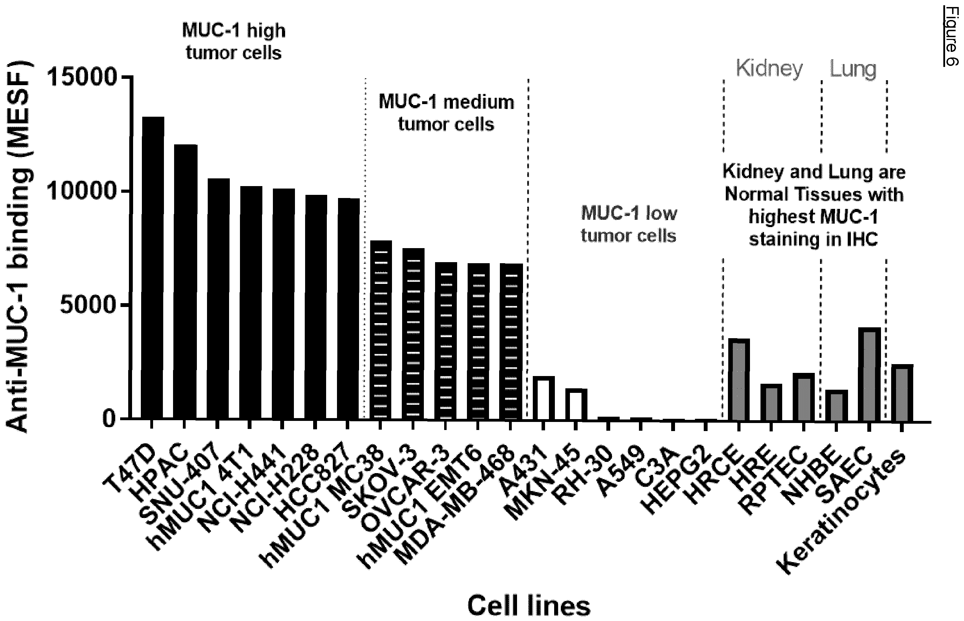

### Example 7: MUC-1 expression on tumor cell lines and normal tissue cell lines (Fig.6)

Primary cells from kidney (Human Renal Cortical Epithelial Cells, Human Renal Proximal Tubule Epithelial Cells, Human Renal Epithelial Cells) and lung (Human Small Airway Epithelial Cells, Human Bronchial Epithelial Cells) were purchased from Lonza and maintained with culturing conditions provided by the supplier. The panel of tumor cells (T47D, SNU047, HPAC, NCI-H441, NCI-H228, HCC827, MDA-MB-468, OVCAR3, SKOV3, MKN45, A549m A431, RH-30, C3A) was provided by Merck Darmstadt Cell Bank and maintained with RPMI1640 (PAN Biotech) or DMEM (PAN Biotech) based cell-culture media supplemented with 10% FCS (PAN Biotech), 10 mM L-Glutamine, 1x Penicillin/Streptomycin. Aforementioned cell lines were enzymatically detached with accutase (PAN Biotech), and 100 000 cells seeded into each 96 well of a V-bottom plate. Cells were washed, blocked with beriglobin (Behring CSL), and stained with anti-MUC-1 (D11-28) PE-labeled antibody. Stained cells were measured in an LSR II Fortessa (BD Biosciences). PE Quantum Beads (Bangs Laboratories) were used to quantify MESF (Molecules of Equivalent Soluble Fluorochrome) values after anti-MUC-1 PE antibody binding to each cell line using bead manufacturers standard protocol. The graph displays the quantity of anti-MUC-1 PE antibody as Molecules of Equivalent Soluble Fluorochrome (MESF) from tumor cell lines and normal kidney and normal lung cells.

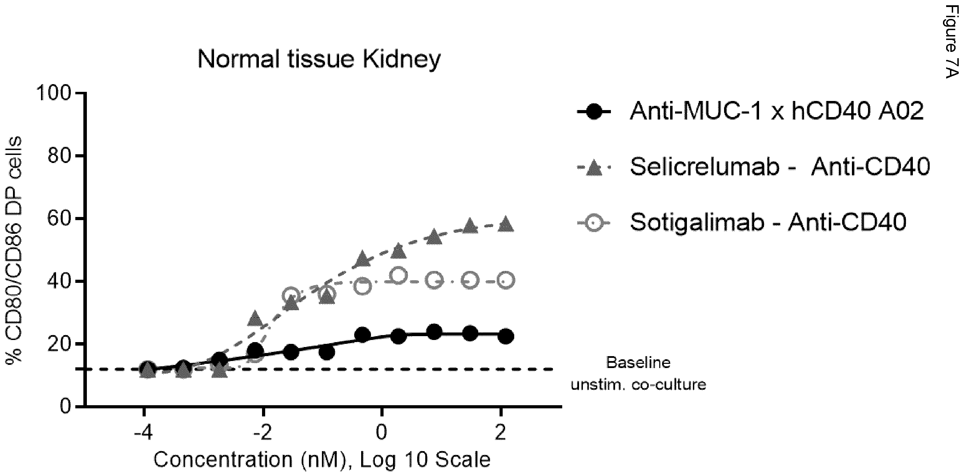

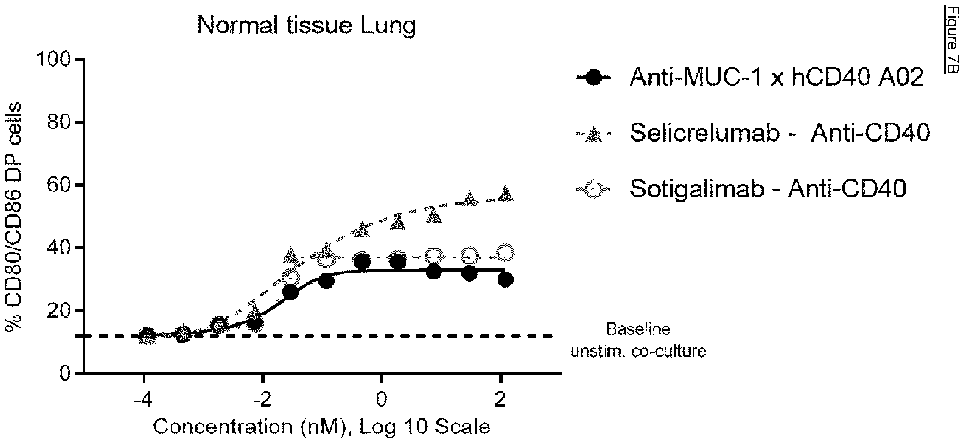

### Example 8: Low activation of CD40 by anti-MUC-1 × hCD40 A02 using normal tissue Kidney and Lung (Fig. 7)

Human peripheral blood mononuclear cells (PBMCs) were purified out of whole blood from healthy donors using a ficoll gradient (Pancoll human / PAN Biotech). After washing of cells using Dulbecco's PBS (PAN Biotech), PBMCs were counted, and a cell suspension prepared for magnetic cell separation. Human monocyte Enrichment Kit without CD16 Depletion (Stemcell Technologies) was used to isolate untouched monocytes out of PBMCs via negative selection. Isolated monocytes were counted and adjusted for differentiation into monocyte-derived immature dendritic cells using 200 ng/ml GM-CSF and 50 ng/ml IL-4 (both Peprotech) in RPMI1640 medium supplemented with 10% Fetal Bovine Serum (PAN Biotech), 2 mM L-Glutamine (PAN Biotech), 1x Penicillin/Streptomycin (PAN Biotech). Primary cells from kidney (Human Renal Cortical Epithelial Cells, Human Renal Proximal Tubule Epithelial Cells, Human Renal Epithelial Cells) and lung (Human Small Airway Epithelial Cells, Human Bronchial Epithelial Cells) were purchased from Lonza and maintained with culturing conditions provided by the cell supplier (Lonza). Primary cells were counted (50.000 cells per 96 U-bottom well) and co-cultured with monocyte-derived immature dendritic cells (50.000 cells per 96 U-bottom well). The co-cultures were treated for 24 hours and CD40 activation assessed through CD80/CD86 staining. Briefly, dendritic cells in suspension were washed, blocked with beriglobin (Behring CSL) and stained with dendritic cell markers for lineage identification (CD45+, CD1a+, both from Invitrogen) and also anti-CD80 PE (BD Biosciences) and anti-CD86 FITC (Biolegend) fluorochrome labeled flow cytometry antibodies. Stained cells were washed and fixed with 4% paraformaldehyde (Thermo Fischer Scientific) and measured in an LSR II Fortessa (BD Biosciences). CD45+ and CD1a+ dendritic cells were gated out of the total population. The percentage of CD80 and CD86 double-positive dendritic cells are displayed in the figure.

As can be seen from Fig. 7, anti-MUC-1 × CD40 causes only very low levels of CD40 activation in normal kidney and lung cells. In this example, selicrelumab and sotigalimab caused higher levels of CD40 activation in normal kidney and lung cells.

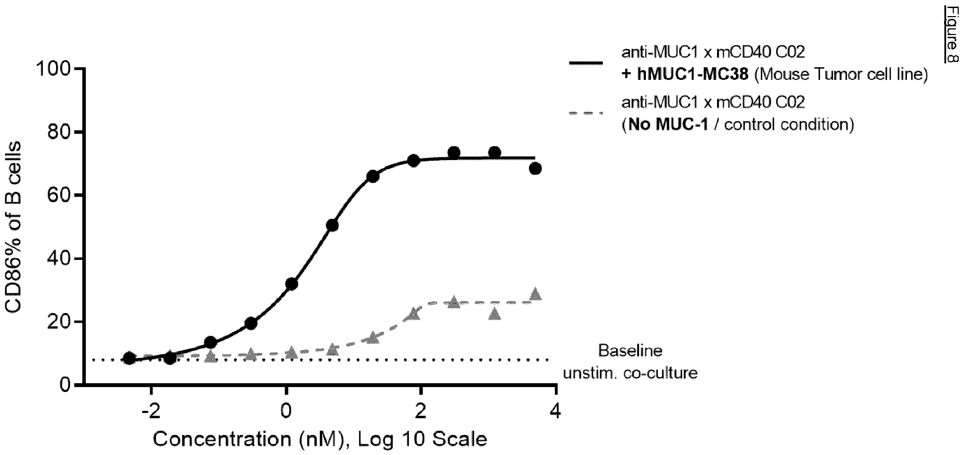

### Example 9: In vitro activity of Anti-MUC-1 × mCD40 C02 mouse surrogate molecule (Fig. 8)

A surrogate anti-mouse CD40 VHH was prepared. To this end, the heavy chain nucleotide sequence of the anti-MUC-1-anti-hCD40-VHH was adapted for use as a surrogate in mouse (i.e. anti-MUC-1-anti-mCD40-VHH heavy chain was generated) and cloned into an expression vector. Similarly, the nucleotide sequence of the anti-MUC-1 lambda light chain was adapted for use as a surrogate in mouse and cloned into an expression vector. In both cases, codon usage was optimized for mammalian expression.

Mouse spleens were isolated from C57/BL6 mice and single cell suspensions generated using a cell strainer (BD Biosciences) and a plunger to mesh the spleen. Cells were resuspended with ACK lysis buffer (Thermo Fischer Scientific) to lyse red blood cells. Mouse B cells were isolated out of splenocytes using an Easysep mouse B cell isolation kit (Stemcell technologies). MC38 expressing tumor cells were generated via transfection of exogenous human MUC-1 expressing plasmid into MC38 parental cells. Single cell clones from transfected MC38 cells were isolated to yield hMUC-1 overexpressing MC38 cells and stable transfection of hMUC-1 verified after weeks of maintenance. A clone was selected with stable hMUC-1 overexpression. hMUC-1 expressing MC38 cells (50 000 cells per 96 well U-bottom) were co-cultured with isolated mouse B cells (50 000 cells per 96 well U-bottom). Co-cultures of hMUC-1 MC38 and mouse B cells or monocultures of mouse B cells without hMUC-1 expressing MC38 tumor cells were treated for 24 hours with increasing doses of anti-MUC-1 × mCD40 C02. Cells in suspension were collected in a 96wV-bottom plate and CD86 expression was analyzed by flow cytometry. Briefly, mouse B cells were washed, blocked with beriglobin (Behring CSL) and stained with mouse B cell markers for lineage identification (CD19, Biolegend and B220 BD Biosciences) and also anti-CD86 FITC (BD Biosciences) fluorochrome labeled flow cytometry antibodies. Stained cells were washed and fixed with 4% paraformaldehyde (Thermo Fischer Scientific) and measured in an LSR II Fortessa (BD Biosciences). CD19+ B cells were gated out of the total population. The percentage of CD86 positive B cells after anti-MUC-1 × mCD40 treatment is displayed in the figure showing magnitude of B cell activation after anti-MUC-1 × mCD40 treatment in the presence or absence of hMUC-1 MC38 cells.

As can be seen from Fig. 8, the mouse surrogate anti MUC-1 × mCD40 shows conditional activation of CD40 (and thus, B cells) only in the presence of tumor cells overexpressing MUC-1, but not in the absence of such cells, and thus behaves as the human molecule anti MUC-1 × hCD40.

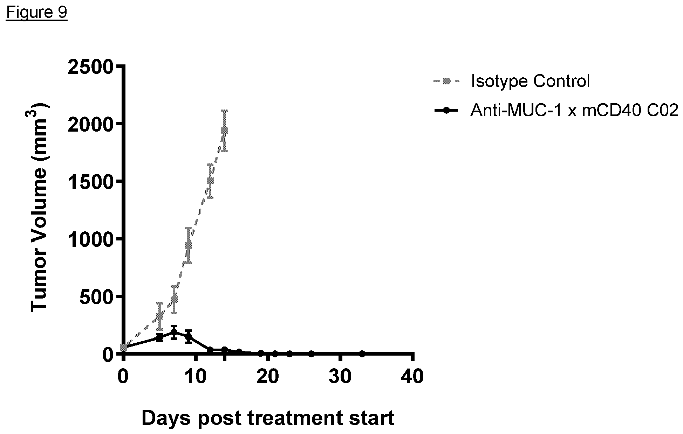

### Example 10: In vivo activity of anti-MUC-1 × mCD40 C02 mouse surrogate in MUC-1-MC38 tumor model (Fig. 9)

MUC-1 expressing tumor cells were generated via lentiviral transduction of exogenous human MUC-1 expressing plasmid into MC38 parental cells. Single cell clones from transduced MC38 cells were isolated to yield hMUC-1 high overexpressing MC38 cells and stable transfection of hMUC-1 verified after eight weeks of maintenance. A clone was selected with high stable hMUC-1 overexpression. The C57BL/6 mice strain was used as host and hMUC-1 overexpressing MC38 tumor cells implanted subcutaneously into the flank of mice. Tumor volume (mm³) was followed up at least once weekly using a caliper and tumor volume calculation was done by calculating measured length × width × width/2. Six days after tumor injection, mice were randomized into treatment groups and intravenously treated with anti-MUC-1 × mCD40 (7.5 mg/kg) single dose or with anti-MUC-1 × mCD40 (7.5 mg/kg) repeat dose at d0, d14, d28. An isotype control was intravenously applied as a third group using a non-mouse CD40 binding anti-MUC-1 × hCD40 A02 molecule.

As can be seen from Fig. 9, anti MUC-1 × mCD40 shows strong *in vivo* activity in the MUC-1-MC38 tumor model for colorectal cancer.

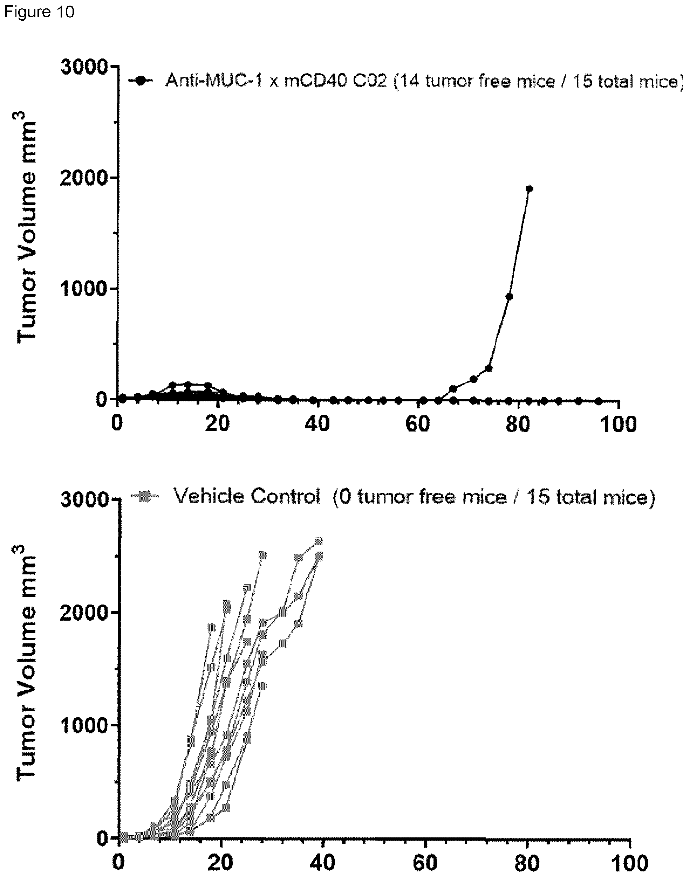

### Example 11: In vivo activity of anti-MUC-1 × mCD40 C02 mouse surrogate in MUC-1-Panc02 tumor model (individual tumor growth, Fig. 10)

MUC-1 expressing Panc02 tumor cells were generated via lentiviral transduction of exogenous human MUC-1 expressing plasmid into Panc02 parental cells. Single cell clones from transduced Panc02 cells were isolated to yield hMUC-1 high overexpressing Panc02 cells and stable transfection of hMUC-1 verified after weeks of maintenance. A clone was selected with high stable hMUC-1 overexpression. Two million hMUC-1 Panc02 tumor cells were orthotopically inoculated into pancreas of non-tumor bearing C57BL/6 mice. The tumor volume of the primary tumor was estimated by palpating the tumor and measuring its diameter using a caliper. The volume of the tumor was calculated using the formula for spherical volume (4/3 × pi × radius3). Mice were distributed into treatment groups and treatment started 7 days after tumor cell injection. Treatment was intravenously applied by injection of anti-MUC-1 × mCD40 C02 (5 mg/kg) or injection of isotype control (in PBS based vehicle). The graph shows tumor volume development over time after single dose anti-MUC-1 × mCD40 C02 injection or after vehicle control injection.

As can be seen from Fig. 10, anti MUC-1 × mCD40 shows strong *in vivo* activity in the MUC-1-Panc02 tumor model for pancreatic cancer.

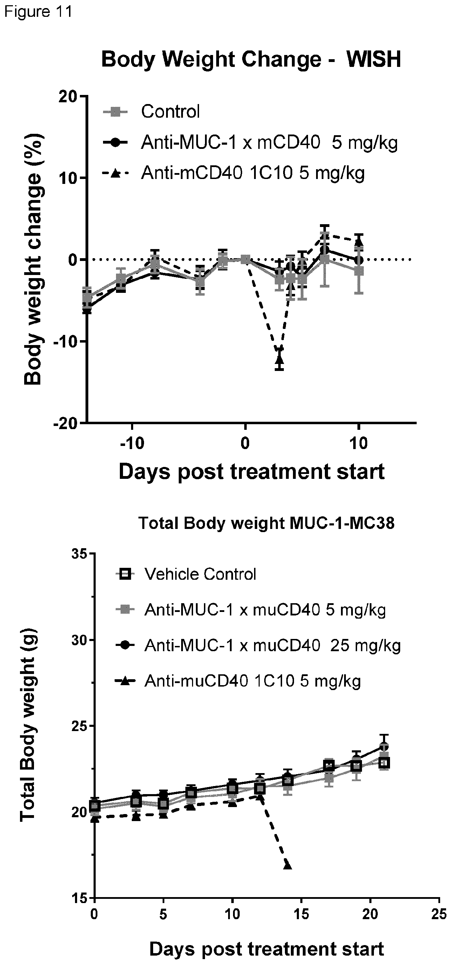

### Example 12: Anti-MUC-1 × mCD40 C02 Mouse Surrogate (Fig. 11)

Human Wish cells (3 million / animal) were subcutaneously implanted into the flank of athymic nude mice (Charles River). Randomization of study took place at tumor sizes of 39-49 mm3. Body weight (g) was followed up at least twice weekly using an animal balance. The body weight change (%) was assessed by deducting body weight pre-treatment from body weight post-treatment and dividing the difference by pre-treatment body weight. Six days after tumor injection, athymic nude mice were intravenously treated with anti-MUC-1 × mCD40 (5 mg/kg) or with anti-mCD40 1C10 clone (5 mg/kg). A vehicle control was intravenously applied as a third group using drug buffer (PBS based buffer). Body weight development is displayed in the graph to indicate detrimental effects of treatments after body weight loss.

MUC-1 expressing tumor cells were generated via transfection of exogenous human MUC-1 expressing plasmid into MC38 parental cells. Single cell clones from transfected MC38 cells were isolated to yield hMUC-1 overexpressing MC38 cells and stable transfection of hMUC-1 verified after weeks of maintenance. A clone was selected with high stable hMUC-1 overexpression. The C57BL/6 mice strain was used as host and hMUC-1 overexpressing MC38 tumor cells (0.75 million per mice) implanted subcutaneously into the right flank of mice. Body weight (g) was followed up at least twice weekly using an animal balance. Twelve days after tumor injection, mice were randomized into treatment groups and intravenously treated with anti-MUC-1 × mCD40 (5 mg/kg) or with anti-mCD40 1C10 clone (5 mg/kg). An isotype control was intravenously applied as a third group using a non-mouse CD40 binding anti-MUC-1 × hCD40 A02 molecule. A vehicle control was intravenously applied as a fourth group using drug buffer. A 2nd treatment was done 5 days later. Body weight development is displayed in the graph to indicate detrimental effects of treatments after body weight loss.

### REFERENCES

Chen W et al., MUC1: Structure, Function, and Clinic Application in Epithelial Cancers. Int J Mol Sci. 2021;22:6567; https://doi.org/10.3390/ijms22126567.
Chen Y et al., Selection and analysis of an optimized anti-VEGF antibody: Crystal structure of an affinity-matured Fab in complex with antigen. J Mol Biol 1999 Nov 5;293(4):865-881. doi: 10.1006/jmbi.1999.3192.
Clackson T et al., Making antibody fragments using phage display libraries. Nature 1991 Aug 15;352(6336):624-628. doi: 10.1038/352624a0.
Clynes R et al., Fc receptors are required in passive and active immunity to melanoma. Proc Natl Acad Sci U S A 1998 Jan 20;95(2):652-6. doi: 10.1073/pnas.95.2.652.
Cole S et al., Monoclonal Antibodies and Cancer Therapy, Alan R. Liss, Inc. 1985, 77-96.
Elgueta R et al., Molecular mechanism and function of CD40/CD40L engagement in the immune system. Immunol Rev. 2009 May; 229(1): 10.1111/j.1600-065X.2009.00782.x.
Gibbs, W et al., Nanobodies. Scientific American Magazine, 2005 Aug;293:2.
Green LL et al., Antigen-specific human monoclonal antibodies from mice engineered with human Ig heavy and light chain YACs. Nat Genet 1994 May;7(1):13-21. doi: 10.1038/ng0594-13.
Hawkins RE et al., Selection of phage antibodies by binding affinity. Mimicking affinity maturation. J Mol Biol 1992 Aug 5;226(3):889-96. doi: 10.1016/0022-2836(92)90639-2.
Kabat EA et al., Sequences of Immunological Interest, Fifth Edition, National Institute of Health, Bethesda, MD (1991).
Koehler G et al., Continuous cultures of fused cells secreting antibody of predefined specificity. Nature, 1975 Aug 7;256: 495-497.
Kontermann RE and Dübel S, Antibody Engineering. Springer; 2nd ed. 2010 Edition (March 26, 2010). ISBN-10: 3662495945; ISBN-13: 978-3662495940.
Kozbor D et al., The production of monoclonal antibodies from human lymphocytes. Immunology Today 1983 Mar;4(3):72-79. doi: 10.1016/0167-5699(83)90123-8.
Ligtenberg MJ et al., Cell-associated episialin is a complex containing two proteins derived from a common precursor. The Journal of Biological Chemistry 1992 March 25;. 267 (9): 6171-6177.
Little M, Recombinant Antibodies for Immunotherapy, Cambridge University Press, 1st ed. (October 8 2009). ISBN-10: 0521887321; ISBN-13: 978-0521887328.
Lowman HB et al., Selecting high-affinity binding proteins by monovalent phage display. Biochemistry 1991 Nov 12;30(45):10832-8. doi: 10.1021/bi00109a004.
Lund J et al., Human Fc gamma RI and Fc gamma RII interact with distinct but overlapping sites on human IgG.J Immunol. 1991 Oct 15;147(8):2657-2662.
Malmborg AC et al., BIAcore as a tool in antibody engineering. J Immunol Methods 1995 Jun 14;183(1):7-13. doi: 10.1016/0022-1759(95)00018-6.
Marks JD et al., By-passing immunization. Human antibodies from V-gene libraries displayed on phage. J Mol Biol 1991 Dec 5;222(3):581-597. doi: 10.1016/0022-2836(91)90498-u.
Ravetch JV et al., Fc receptors. Annu Rev Immunol 1991:9:457-492. doi: 10.1146/annurev.iy.09.040191.002325.
Salomon R and Dahan R, Next Generation CD40 Agonistic Antibodies for Cancer Immunotherapy. Front Immunol. 2022 Jul 13:13:940674. doi: 10.3389/fimmu.2022.940674. eCollection 2022.
Schier R et al., Efficient in vitro affinity maturation of phage antibodies using BIAcore guided selections Human Antibodies 1996, vol. 7, no. 3, pp. 97-105.
Smith GP, Filamentous fusion phage: novel expression vectors that display cloned antigens on the virion surface. Science 1985 Jun 14;228(4705):1315-1317. doi: 10.1126/science.4001944.
US 2003/0070185A1
U.S. Patent No. 4,816,567
U.S. Patent No. 5,223,409
US Patent 5,648,260
WO 96/34096
WO 96/33735

## Claims

1. A bispecific binding protein binding MUC-1 and CD40.

2. The bispecific binding protein of claim 1, wherein the bispecific binding protein comprises an anti-MUC-1 antibody or an anti-MUC-1 antibody fragment and/or one, two or more anti-CD40 VHHs.

3. The bispecific binding protein of claim 2, wherein the an anti-MUC-1 antibody or the anti-MUC-1 antibody fragment comprises a VH CDR1 amino acid sequence according SEQ ID NO: 1, a VH CDR2 amino acid sequence according to SEQ ID NO: 2 and a VH CDR3 amino acid sequence according to SEQ ID NO: 3; and a VL CDR1 amino acid sequence according SEQ ID NO: 6, a VL CDR2 amino acid sequence according to SEQ ID NO: 7 and a VL CDR3 amino acid sequence according to SEQ ID NO: 8.

4. The bispecific binding protein of claim 2 or claim 3, wherein the anti-CD40 VHH comprises a CDR1 amino acid sequence according to SEQ ID NO: 12, a CDR2 amino acid sequence according to SEQ ID NO: 13 and a CDR3 amino acid sequence according to SEQ ID NO: 14.

5. The bispecific binding protein of any one of claims 2-4, wherein the anti-MUC-1 antibody or the MUC-1 antibody fragment comprises
(a) a variable heavy chain amino acid sequence having at least 80% sequence identity with SEQ ID NO: 4 and a variable light chain amino acid sequence having at least 80% sequence identity with SEQ ID NO: 9,
(b) a variable heavy chain amino acid sequence having at least 90% sequence identity with SEQ ID NO: 4 and a variable light chain amino acid sequence having at least 90% sequence identity with SEQ ID NO: 9,
(c) a variable heavy chain amino acid sequence having at least 95% sequence identity with SEQ ID NO: 4 and a variable light chain amino acid sequence having at least 95% sequence identity with SEQ ID NO: 9, or
(d) a variable heavy chain amino acid sequence having at least 99% sequence identity with SEQ ID NO: 4 and a variable light chain amino acid sequence having at least 99% sequence identity with SEQ ID NO: 9,
preferably wherein the bispecific binding protein exhibits at least 80%, at least 90% or at least 95% of the binding activity of an anti-MUC-1 antibody with a variable heavy chain amino acid sequence of SEQ ID NO: 4 and a variable light chain amino acid sequence of SEQ ID NO: 9.

6. The bispecific binding protein of any one of claims 2-5, wherein the anti-CD40 VHH comprises an amino acid sequence having at least 80%, at least 90%, at least 95% or at least 99% sequence identity with SEQ ID NO: 15,
preferably wherein the bispecific binding protein exhibits at least 80%, at least 90% or at least 95% of the binding activity of an anti-CD40 VHH with an amino acid sequence SEQ ID NO: 15.

7. The bispecific binding protein of any one of claims 2-6, wherein the anti-MUC-1 antibody or the MUC-1 antibody fragment and the anti-CD40 VHH are directly liked in a fusion protein with an amino acid linker sequence preferably wherein the amino acid linker sequence comprises 10-30 amino acids and/or wherein the amino acids are selected from G and S, more preferably, wherein the amino acid linker sequence comprises an amino acid sequence according

8. The bispecific binding protein of any one of claims 2-7, wherein the anti-CD40 VHH is linked to the C-terminus of the anti-MUC-1 antibody or the MUC-1 antibody fragment, preferably wherein the anti-CD40 VHH is linked to the C-terminus of the heavy chain of the anti-MUC-1 antibody.

9. The bispecific binding protein of any one of claims 1-8, wherein the bispecific binding protein comprises a light chain amino acid sequence according to SEQ ID NO: 10 and a heavy chain amino acid sequence according to SEQ ID NO: 16.

10. The bispecific binding protein of any one of claims 1-9, wherein the bispecific binding protein binds to MUC 1 with a dissociation constant (KD) of < 1×10⁻⁶M and/or wherein the bispecific binding protein binds to CD40 with a dissociation constant (KD) of < 1×10⁻⁶M.

11. The bispecific binding protein of any one of claims 1-10, wherein the bispecific binding protein induces CD40 activation on B cells in the presence of MUC-1-expressing tumor cells and/or wherein the bispecific binding protein induces CD40 activation on dendritic cells in the presence of MUC-1-expressing tumor cells.

12. The bispecific binding protein of any one of claims 1-11 for use in therapy.

13. The bispecific binding protein of any one of claims 1-11 for use in the treatment of cancer, optionally wherein the cancer is selected from lung cancer, pancreatic cancer and colorectal cancer.

14. A nucleic acid encoding the bispecific binding protein of any one of claims 1-11.

15. A method for preparing a bispecific protein of any one of claims 1-11.
